(19) Europäisches Patentamt / European Patent Office / Office européen des brevets

(11) **EP 2 332 523 B2**

(12) **NEW EUROPEAN PATENT SPECIFICATION**
After opposition procedure

(45) Date of publication and mention
of the opposition decision:
**24.11.2021 Bulletin 2021/47**

(45) Mention of the grant of the patent:
**11.09.2013 Bulletin 2013/37**

(51) Int Cl.:
*A61K 31/4545* (2006.01)   *A61K 9/00* (2006.01)
*A61K 9/70* (2006.01)   *A61K 9/16* (2006.01)
*A61K 45/00* (2006.01)

(21) Application number: **11157819.1**

(22) Date of filing: **11.10.2002**

(54) **UNIFORM FILMS FOR RAPID DISSOLVE DOSAGE FORM INCORPORATING TASTE-MASKING COMPOSITIONS**

EINHEITLICHE FILME ALS SCHNELL AUFLÖSENDE VERABREICHUNGSFORMEN ENTHALTEND GESCHMACKSMASKIERENDEN STOFF

Films uniformes pour la forme de dosage de dissolution rapide incorporant des compositions de masquage du goût

(84) Designated Contracting States:
**AT BE BG CH CY CZ DE DK EE ES FI FR GB GR IE IT LI LU MC NL PT SE SK TR**

(30) Priority: **12.10.2001 US 328868 P**
**14.02.2002 US 74272**
**07.06.2002 US 386937 P**
**27.09.2002 US 414276 P**

(43) Date of publication of application:
**15.06.2011 Bulletin 2011/24**

(62) Document number(s) of the earlier application(s) in
accordance with Art. 76 EPC:
**02801042.9 / 1 458 367**

(73) Proprietor: **Aquestive Therapeutics, Inc.**
**Warren, NJ 07059 (US)**

(72) Inventors:
• **Fuisz, Richard C.**
**BEVERLY HILLS, CA CA 90210 (US)**
• **Yang, Robert K.**
**FLUSHING, NY NY 11355 (US)**
• **Myers, Gary L.**
**KINGSPORT, TN 37660 (US)**

(74) Representative: **Haseltine Lake Kempner LLP**
**Redcliff Quay**
**120 Redcliff Street**
**Bristol BS1 6HU (GB)**

(56) References cited:
EP-A- 0 241 178   WO-A-00/42992
WO-A-01/70194   US-A- 4 136 145
US-A- 4 631 837   US-A- 5 028 632
US-A- 5 567 431   US-A- 6 153 210
US-A1- 2001 006 677

EP 2 332 523 B2

**Description**

## FIELD OF THE DISCLOSURE

The present disclosure relates to compositions and methods for the preparation and use of a uniform rapid dissolve dosage form in the form of a film that includes a pharmaceutically active or bioeffecting agent and a taste-masking agent for masking the taste of the pharmaceutically active agent.

## BACKGROUND OF RELATED TECHNOLOGY

While active ingredients such as pharmaceutical preparations may be included in a tablet or similar form to provide an accurate and consistent dose, including medicaments in such a form has several disadvantages in both the administration and preparation of the drug. Moreover, in such oral dosage forms, such as tablets or emulsions, pharmaceuticals have been coated to provide control release or taste-masking. Particle sizes of particulate pharmaceuticals are not critical in such dosage forms and generally large particle sizes, i.e., greater than 200 microns have been used.

There have been several attempts to provide an alternate dosage form, such as a film that would include a pharmaceutical active. However, such attempts have not been successful in providing a film that incorporates a drug with sufficient uniformity to provide accurate dosing.

Films that incorporate a pharmaceutically active ingredient are disclosed in expired U.S. Patent No. 4,136,145 to Fuchs, et al. ("Fuchs"). These films may be formed into a sheet, dried and then cut into individual doses. The Fuchs disclosure alleges the fabrication of a uniform film, which includes the combination of water-soluble polymers, surfactants, flavors, sweeteners, plasticizers and drugs. These allegedly flexible films are disclosed as being useful for oral, topical or enteral use. Examples of specific uses disclosed by Fuchs include application of the films to mucosal membrane areas of the body, including the mouth, rectal, vaginal, nasal and ear areas.

Examination of films made in accordance with the process disclosed in Fuchs, however, reveals that such films suffer from the aggregation or conglomeration of particles, i.e., self-aggregation, making them inherently non-uniform. This result can be attributed to Fuchs' process parameters, which although not specifically disclosed likely include the use of relatively long drying times, thereby facilitating intermolecular attractive forces, convection forces, air flow and the like to form such agglomeration.

The formation of agglomerates randomly distributes the film components and any active present as well. When large dosages are involved, a small change in the dimensions of the film would lead to a large difference in the amount of active per film. If such films were to include low dosages of active, it is possible that portions of the film may be substantially devoid of any active. Since sheets of film are usually cut into unit doses, certain doses may therefore be devoid of or contain an insufficient amount of active for the recommended treatment. Failure to achieve a high degree of accuracy with respect to the amount of active ingredient in the cut film can be harmful to the patient. For this reason, dosage forms formed by processes such as Fuchs, would not likely meet the stringent governmental or agency standards relating to the variation of active in dosage forms. Currently, by law, dosage forms may not vary more than 10% in the amount of active present. When applied to dosage units based on films, this virtually mandates that uniformity in the film be present.

Moreover, the problems of self-aggregation leading to non-uniformity of a film can result in an unpleasant tasting film when the film contains an unpleasant tasting pharmaceutical agent. Agglomerates of unpleasant tasting pharmaceutical agents may not be adequately masked by flavoring agents and sweeteners that are simply mixed into a film because the non- uniformity of the agglomerates may result in segregation of the unpleasant tasting agents from the flavoring agents and sweeteners. Fuchs merely mixes flavors and sweeteners into a film forming mix and fails to address the problem of aggregation or segregation of these materials.

Similarly, WO 00/42,992 also discloses the use of taste-modifying agents in a film dosage form. This international application also merely mixes taste- modifying agents into the film-forming mix without recognizing the problem of separation or aggregation of the taste-modifying agents from the unpleasant tasting pharmaceutical agents.

Furthermore, WO 01/70,194 discloses the use of ion exchange resins to for covalently binding pharmaceutical agents thereto. The resins have particle sizes from 20 microns to 200 microns and are described as being taste masking agents. The ion exchange resins are described as being bound with pharmaceutical agents and being mixed into consumerable films having thicknesses from 7 to 11 mils, or 180 microns to 280 microns. Such ion exchange resins, however, have limitations in the binding of pharmaceutical agents to the ion exchange resins, making the process for producing taste-masked comsumerable films complicated and expensive. Moreover, the use of ion exchange resins, which are water insoluble, limits the selection of useful pharmaceutical agents in water soluble films to only certain water soluble pharmaceutical agents that can covalently bond to the ionic resin.

Therefore, there is a need for a rapid dissolve dosage form, presented as a uniform film that addresses and corrects the problems associated with non-uniformity of a drug in film such as agglomeration or separation of particles within the film and the unpleasant tasting effects of the same. Moreover, there is a need for taste-masked, pharmaceutically active agents suitably contained within such a uniform film.

## SUMMARY OF THE INVENTION

The present invention seeks to attain low adjuvant content, high taste-masked pharmaceutical active content films which have enhanced flexibility, structural integrity and uniformity. The present disclosure also provides for a unique method of producing compositions such that the compositional components are evenly distributed throughout the film. This process is described in detail in co-pending U.S. Patent Application No. 10/074,272, entitled "Thin Film with Non-Self-Aggregating Uniform Heterogeneity and Drug Delivery Systems Made Therefrom". The present invention is provided according to the appended claims.

Described herein is a drug delivery composition which includes (i) a flowable water-soluble film forming matrix; (ii) a particulate bioeffecting agent uniformly stationed therein; and (iii) a taste-masking agent coated or intimately associated with the particulate to provide taste-masking of the bioeffecting agent. The combined particulate and taste-masking agent have a particle size of 200 microns or less, and the flowable water-soluble film forming matrix is capable of being dried without loss of uniformity in the stationing of the particulate bioeffecting agent therein. The importance of such particle sizes has not been recognized in the prior art, especially in prior art dosage forms, such as tablets and emulsions. Moreover, the importance of particle size is heightened in orally ingestible thin films, where uniformity is also of particular importance, and the prior art has failed to recognize such critically important features.

Desirably, the size of the combined particulate and taste-masking agent have a particle size of 150 microns or less, for example 100 microns or less. Moreover, the flowable water-soluble film forming matrix is formable into a dry film of less than about 380 microns in thickness, for example less than about 250 microns in thickness. Desirably, such particle sizes are contained within these dry films. In other words the dry films described herein desirably have smooth surfaces free of exposed agents that could impart grittiness or maldistribution of the active. Thus, in one aspect described herein there is provided a film vehicle which contains a uniform distribution of actives, as defined herein, being suitably free of particles which accumulate on the film surface when dried.

Desirably, taste-masking agent is a thin film coating over portions of the bioeffecting agent. Useful taste-masking agents include polymeric materials. Water-soluble polymers are also useful. Desirably, the water-soluble polymer has an average molecular weight of equal to or greater than about 40, 000. Furthermore, water-soluble polymers may be acrylic polymers, cellulosic polymers, and combinations thereof. Additionally, vinyl polymers, crown ethers, hydrogenated oils and waxes, and combinations thereof may also be used as taste-masking agents.

The matrix may be a cellulosic material; a gum; a protein; a starch; a glucan; and combinations thereof; such as but not limited to carboxymethyl cellulose; methyl cellulose; hydroxymethyl cellulose; hydroxyethyl cellulose; hydroxypropyl cellulose; hydroxypropylmethyl cellulose; hydroxymethylpropyl cellulose; gum arabic; xanthan gum; tragacanth; acacia; carageenan; guar gum; locust bean gum; pectin; alginates; gelatinized, modified or unmodified starch, including tapioca starch, rice starch, corn starch, potato starch, and wheat starch; polyvinyl alcohol; polyacrylic acid; polyvinyl pyrrolidone; poly(meth)acrylate; poly(meth)copolymers; dextrin; dextran; proteins, such as, gelatin, zein, gluten, soy protein, soy protein isolate, and whey protein; whey protein isolate; casein; levin; collagen; chitin; chitosin; polydextrose and combinations thereof.

The bioeffecting agent may be present in amounts of up to about 0.1% to about 60% by weight of the total composition. Useful bioeffecting agents include, but are not limited to, antimicrobial agents, non-steroidal anti-inflammatory drugs, anti-tussives, decongestants, antihistamines, expectorants, anti-diarrheals, $H_2$ antagonists, proton pump inhibitors, general non-selective CNS depressants, general non-selective CNS stimulants, selective CNS functional modifiers, anti-parkinsonism drugs, narcotics, analgesics, anti-pyretics, psychopharmacological drugs and combinations thereof. The delivery vehicle composition may further include an organoleptic agent.

Described herein is a drug delivery vehicle which includes (i) a water-soluble film matrix; and (ii) a particulate bioeffecting agent uniformly suspended within the matrix and having associated with it a taste-masking agent. The uniformity is determined by the presence of no more than a 10% by weight of drug variance throughout the matrix. Desirably, the drug variance is less than 5% by weight, less than 2% by weight, less than 1% by weight, or less than 0.5% by weight. Moreover, the particulates have a particle size of 200 microns or less. Furthermore, the film matrix desirably has a thickness of less than about 380 microns. Useful taste-masking agents include water-soluble polymers. Desirably, the water-soluble polymer has an average molecular weight of equal to or greater than about 40,000. Non-limiting water-soluble polymers include acrylic polymers, cellulosic polymers, and combinations thereof. The taste-masking agents may also include vinyl polymers, crown ethers, hydrogenated oils and waxes, and combinations thereof. The drug delivery vehicle of claim may further include an organoleptic agent with the bioeffecting agent.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent coated or encapsulated with a water-soluble polymer having an average molecular weight of equal to or greater than about 25,000. Water-soluble polymers having an average molecular weight of equal to or greater than about 40,000 are also useful. Useful water-soluble polymers include of acrylic polymers, cellulosic polymers, and combinations thereof. Desirably, the pharmaceutically active particles are embedded within the film. Additionally, the film includes sections of substantially equal size and the particles are distributed in an amount that varies less than about 10% among the sections. Desirably, the size of the particles are about 200 microns or less. Desirably, the film has a thickness of less than about 380 microns. Moreover, the drug delivery vehicle may further include an organoleptic agent with the water-soluble polymer.

Described herein is a drug delivery vehicle which includes a dry mucoadhering film having a thickness defined by opposed surfaces. The film includes (i) a water-soluble polymer; and (ii) a pharmaceutically active particle having a pharmaceutically active agent and a taste-masking agent present in the amount of about 15-80% by weight of the particle. Desirably, the taste-masking agent is present in the amount of about 20-60% by weight of the particle. More desirably, the taste-masking agent is present in the amount of about 25-35% by weight of the particle. The pharmaceutically active particle is desirably embedded within the film, and the film includes sections of substantially equal size where the particles are distributed in an amount that varies less than about 10% among the sections. Useful sizes of the pharmaceutically active particles include particle sizes of 200 microns or less. Desirably, the film has a thickness of less than about 380 microns. The drug delivery vehicle may further include an organoleptic agent with the taste-masking agent.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent and a taste-masking agent. The active particle has a particle size of less than about 200 microns. Desirably, the thickness of the film is less than about 380 microns.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent and a taste-masking agent. The particle desirably has a particle size of less than about 200 microns and the taste-masking agent is present in amounts of about 15-80% by weight of the particle. A particle size of about 150 microns or less is also useful. Desirably, the particle size of the particle is about 100 microns or less. Desirably, the thickness of the film is less than about 380 microns, for example, less than about 250 microns. Furthermore, the taste-masking agent may be present in the amount of about 20-60% by weight of the particle. Desirably, the taste-masking agent is present in the amount of about 25-35% by weight of the particle.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent and an organoleptic agent. The active particle is taste-masked with a taste-masking agent. Useful organoleptic agents include flavors, sweeteners and combinations thereof.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent being taste-masked with a taste-masking composition comprising a water-soluble polymer and at least one of a flavor or a sweetener.

Described herein is also a method of preparing a thin film drug delivery vehicle The method includes the steps of (a) providing a pharmaceutically active agent / taste-masking agent complex; (b) combining the complex with a water-soluble polymer and a solvent to form a mixture with uniform distribution of the complex therein; (c) casting the mixture onto a planar carrier surface to form a thin film on the carrier surface; and (d) controllably drying the thin film to form a distribution variance of the complex having less than about 10% variance throughout any given area of the thin film. The step of providing the pharmaceutically active agent with the taste-masking agent includes a treatment for coating the taste masking agent onto portions of the pharmaceutically active agent. The drying includes applying heat to the bottom of the carrier surface. Moreover, the drying may include applying microwave energy to the film. Useful methods for providing the pharmaceutically active agent with the taste-masking agent include fluidized bed coating, spray congealing coating, agglomeration or granulation coating, entrapment coating, coaccervation coating, infusion coating, spin coating, and ion exchange coating the taste masking agent onto portions of the pharmaceutically active agent.

## BRIEF DESCRIPTION OF THE DRAWINGS

Figure 1 shows a side view of a package containing a unit dosage film.

Figure 2 shows a top view of two adjacently coupled packages containing individual unit dosage forms separated by a tearable perforation.

Figure 3 shows a side view of the adjacently coupled packages of Figure 2 arranged in a stacked configuration.

Figure 4 shows a perspective view of a dispenser for dispensing the packaged unit dosage forms, dispenser containing the packaged unit dosage forms in a stacked configuration.

Figure 5 is a schematic view of a roll of coupled unit dose packages.

Figure 6 is a schematic view of an apparatus suitable for preparation of a pre-mix, addition of an active, and subsequent formation of the film.

Figure 7 is a schematic view of an apparatus suitable for drying the films.

## DETAILED DESCRIPTION

Described herein is a pharmaceutical composition in the form of a film for external or topical administration, including a composition having a uniformly distributed combination of a polymer, a polar solvent, and a taste-masked pharmaceutically active or bioeffecting agent. The composition in its dried film form maintains the uniform distribution of components through the application of controlled bottom drying of the film.

Water-soluble polymers useful in the present disclosure include cellulosic materials, gums, proteins, starches, and combinations thereof.

As used herein the phrase "water soluble polymer" and variants thereof refer to a polymer that is at least partially soluble in water, and desirably fully or predominantly soluble in water, or absorbs water. Polymers that absorb water are often referred to as being water swellable polymers. The materials useful with the present invention may be water soluble or water swellable at room temperature and other temperatures, such as temperatures exceeding room temperature. Moreover, the materials may be water soluble or water swellable at pressures less than atmospheric pressure. Desirably, the water soluble polymers are water soluble or water swellable having at least 20 percent by weight water uptake. Water swellable polymers having a 25 or greater percent by weight water uptake are also useful. Films or dosage forms of the present invention formed from such water soluble polymers are desirably sufficiently water soluble to be dissolvable upon contact with bodily fluids.

Examples of cellulosic materials include, without limitation, carboxymethyl cellulose, methyl cellulose, ethyl cellulose, hydroxymethyl cellulose, hydroxyethyl cellulose, hydroxypropyl cellulose, hydroxypropylmethyl cellulose, hydroxymethylpropyl cellulose, and combinations thereof.

Examples of water-soluble gums include gum arabic, xanthan gum, tragacanth, acacia, carageenan, guar gum, locust bean gum, pectin, alginates and combinations thereof.

Examples of other polymeric materials which may be incorporated include polyvinyl alcohol, polyacrylic acid, polyvinyl pyrrolidone, poly (meth) acrylate, poly (meth) copolymers and combinations thereof.

Useful starches include gelatinized, modified or unmodified starches. The source of the starches may vary and include pullulan, tapioca, rice, corn, potato, wheat and combinations thereof.

Useful water-soluble protein polymers include gelatin, zein, gluten, soy protein, soy protein isolate, whey protein, whey protein isolate, casein, levin, collagen and combinations thereof. Additional water-soluble polymers include dextrin, dextran and combinations thereof, as well as chitin, chitosin and combinations thereof, polydextrose and fructose oligomers.

Although a variety of different polymers may be used, it is desired to select polymers to provide a desired viscosity of the mixture prior to drying. The polymer plays an important role in affecting the viscosity of the film. Viscosity is one property of a liquid that controls the stability of the active in an emulsion, a colloid or a suspension. Generally the viscosity of the matrix will vary from about 400 cps to about 100,000 cps, preferably from about 800 cps to about 60,000 cps, and most preferably from about 1,000 cps to about 40,000 cps. Desirably, the viscosity of the film-forming matrix will rapidly increase upon initiation of the drying process.

The edible water-soluble delivery system described herein further includes glucans, such as pullulan and elsinan. The ratio of glucan to water soluble polymer is about 40:1 to about 0.1:5. Glucans are generally desirable materials for edible film because of their high water solubility, rapid dissolution and excellent mouth-feel.

The edible water-soluble delivery system described herein further includes an anti-foaming or defoaming agent, such as simethicone, which is a combination of a polymethylsiloxane and silicon dioxide. Simethicone acts as either an anti-foaming or defoaming agent which reduces or eliminates air from the film composition. An anti-foaming agent will aid in preventing the introduction of air into a composition, while a defoaming agent will aid in removing air from the composition.

The edible water-soluble delivery system described herein further includes an active component selected from cosmetic agents, pharmaceutical agents, bioactive agents and combinations thereof. The active component may be present in any amount effective for the intended treatment. It is particularly desirable and an advantage of the present invention that the active component can be included in high loads. For example, the active component may be present in amounts up to about 60% by weight of the total composition and desirably in amounts of 0.01 % to about 50% by weight of the total composition.

The pharmaceutically or bioeffecting active components that may be incorporated into the films described herein include a wide variety of medicaments and pharmaceutical compositions. Examples of useful drugs include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, anti-pyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper- and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Erectile dysfunction therapies include, but are not limited to, drugs for facilitating blood flow to the penis, and for effecting autonomic nervous activities, such as increasing parasympathetic (cholinergic) and decreasing sympathetic (adrenersic) activities. Useful non-limiting drugs include sildenafils, such as Viagra®, tadalafils, such as Cialis®, vardenafils, apomorphines, such as Uprima®, yohimbine hydrochlorides such as Aphrodyne®, and alprostadils such as Caverject®.

Examples of medicating active ingredients contemplated for use in the present invention include antacids, $H_2$-antagonists, and analgesics. For example, antacid dosages can be prepared using the ingredients calcium carbonate alone or in combination with magnesium hydroxide, and/or aluminum hydroxide. Moreover, antacids can be used in combination with $H_2$-antagonists.

Analgesics include opiates and opiate derivatives, such as oxycodone (available as Oxycontin®), ibuprofen, aspirin, acetaminophen, and combinations thereof that may optionally include caffeine.

Other preferred drugs for other preferred active ingredients for use in the present invention include anti-diarrheals such as immodium AD, anti-histamines, anti-tussives, decongestants, vitamins, and breath fresheners. Common drugs used alone or in combination for colds, pain, fever, cough, congestion, runny nose and allergies, such as acetaminophen, chlorpheniramine maleate, dextromethorphan, pseudoephedrine HCl and diphenhydramine may be included in the film compositions.

Also contemplated for use herein are anxiolytics such as alprazolam (available as Xanax®); anti-psychotics such as clozopin (available as Clozaril®) and haloperidol (available as Haldol®); non-steroidal anti-inflammatories (NSAID's) such as dicyclofenacs (available as Voltaren®) and etodolac (available as Lodine®), anti-histamines such as loratadine (available as Claritin®), astemizole (available as Hismanal™), nabumetone (available as Relafen®), and Clemastine (available as Tavist®); anti-emetics such as granisetron hydrochloride (available as Kytril®) and nabilone (available as Cesamet™); bronchodilators such as Bentolin®, albuterol sulfate (available as Proventil®); anti-depressants such as fluoxetine hydrochloride (available as Prozac®), sertraline hydrochloride (available as Zoloft®), and paroxtine hydrochloride (available as Paxil®); anti-migraines such as Imigra®, ACE-inhibitors such as enalaprilat (available as Vasotec®), captopril (available as Capoten®) and lisinopril (available as Zestril®); anti-Alzheimer's agents, such as nicergoline; and $Ca^H$-antagonists such as nifedipine (available as Procardia® and Adalat®), and verapamil hydrochloride (available as Calan®).

The popular $H_2$-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts.

The pharmaceutically active agents employed in the present invention may include allergens or antigens, such as , but not limited to, plant pollens from grasses, trees, or ragweed; animal danders, which are tiny scales shed from the skin and hair of cats and other furred animals; insects, such as house dust mites, bees, and wasps; and drugs, such as penicillin.

The pharmaceutically active agents employed in the present invention may be incorporated into the film compositions in a taste-masked form. For example, particles of drug may be coated with taste-masking agents, for example polymers, oils and waxes. Additionally, organoleptic agents, such as, but not limited to sweeteners and/or flavors, may also be employed in such taste-masked compositions, including in the coating layer of the taste masking agent.

Suitable sweeteners include both natural and artificial sweeteners. Non-limiting examples of suitable sweeteners include, e.g.:

a. water-soluble sweetening agents such as monosaccharides, disaccharides and polysaccharides such as xylose, ribose, glucose (dextrose), mannose, galactose, fructose (levulose), sucrose (sugar), maltose, invert sugar (a mixture of fructose and glucose derived from sucrose), partially hydrolyzed starch, corn syrup solids, dihydrochalcones, monellin, steviosides, and glycyrrhizin;

b. water-soluble artificial sweeteners such as the soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (acesulfame-K), the free acid form of saccharin and the like;

c. dipeptide based sweeteners, such as L-aspartic acid derived sweeteners, such as L-aspartyl- L-phenylalanine methyl ester (aspartame), L-alpha-aspartyl-N-(2,2,4,4-tetramethyl-3-thietanyl)-D-alaninamide hydrate, methyl esters of L-aspartyl-L-phenylglycerin and L-aspartyl- L-2,5,dihydrophenylglycine, L-aspartyl-2,5-dihydro-L-phenylalanine, L-aspartyl-L-(1-cyclohexyen)-alanine, and the like;

d. water-soluble sweeteners derived from naturally occurring water-soluble sweeteners, such as a chlorinated derivatives of ordinary sugar (sucrose), known, for example, under the product description of sucralose; and

e. protein based sweeteners such as thaurnatoccous danielli (Thaurnatin I and II).

In general, an effective amount of auxiliary sweetener is utilized to provide the level of sweetness desired for a particular composition, and this amount will vary with the sweetener selected. This amount will normally be 0.01 % to about 10 % by weight of the composition. These amounts may be used to achieve a desired level of sweetness independent from the flavor level achieved from any optional flavor oils used. Of course, sweeteners need not be added to films intended for non-oral administration.

Useful flavors or flavoring agents include natural and artificial flavors. These flavorings may be chosen from synthetic flavor oils and flavoring aromatics, and/or oils, oleo resins and extracts derived from plants, leaves, flowers, fruits and so forth, and combinations thereof. Non-limiting flavor oils include: spearmint oil, cinnamon oil, peppermint oil, clove oil, bay oil thyme oil, cedar leaf oil, oil of nutmeg, oil of sage, and oil of bitter almonds. Also useful are artificial, natural or synthetic fruit flavors such as vanilla, chocolate, coffee, cocoa and citrus oil, including lemon, orange, grape, lime and grapefruit, and fruit essences including apple, pear, peach, strawberry, raspberry, cherry, plum, pineapple, apricot and the like. These flavorings can be used individually or in combination. Commonly used flavors include mints such as peppermint, artificial vanilla, cinnamon derivatives, and various fruit flavors, whether employed individually or in combination. Flavorings such as aldehydes and esters including cinnamylacetate, cinnamaldehyde, citral, diethylacetal, dihydrocarvyl acetate, eugenyl formate, p-methylanisole, and the like may also be used. Further examples of aldehyde flavorings include, but are not limited to acetaldehyde (apple); benzaldehyde (cherry, almond); cinnamicaldehyde (cinnamon); citral, i.e., alpha citral (lemon, lime) ; neral, i.e. beta citral (lemon, lime); decanal (orange, lemon) ; ethyl vanillin (vanilla, cream); haliotropine, i.e., piperonal (vanilla, cream); vanillin (vanilla, cream); alpha-amyl cinnamaldehyde (spicy fruity flavors); butyraldehyde (butter, cheese); valeraldehyde (butter, cheese); citronellal (modifies, many types); decanal (citrus fruits); aldehyde C-8 (citrus fruits); aldehyde C-9 (citrus fruits); aldehyde C-12 (citrus fruits); 2-ethyl butyraldehyde (berry fruits); hexenal, i.e. trans-2 (berry fruits); tolyl aldehyde (cherry, almond); veratraldehyde (vanilla); 12,6-dimethyl-5-heptenal, i.e. melonal (melon); 2 dimethyloctanal (greenfruit); and 2-dodecenal (citrus, mandarin); cherry; grape; mixtures thereof; and the like.

The amount of flavoring employed is normally a matter of preference, subject to such factors as flavor type, individual flavor, and strength desired. The amount may be varied in order to obtain the result desired in the final product. Such variations are within the capabilities of those skilled in the art without the need for undue experimentation. In general, amounts of about 0.1 to about 30 wt% are useful with the practice of the present invention.

A variety of polymeric and non-polymeric materials can be employed for taste masking pharmaceutically active agents. Non-limiting examples of polymers include acrylic polymers, cellulosic polymers or vinyl polymers. Non-limiting examples of non-polymeric materials include crown ethers, fully hydrogenated oils and waxes. Moreover, the taste masking agents may be water soluble, water insoluble or partially water soluble.

Useful non-limiting acrylic polymers include those available under the trade name Eudragit® from Röhm America, LLC, such as methacrylic acid co-polymers sold under the trade names Eudragit E@, Eudragit L@, Eudragit RD® and Eudragit S®, and polyethylacrylate-methylmethacrylate sold under the trade name, Eudragit NE®. These acrylic polymers are generally water soluble materials.

Useful non-limiting cellulosic polymers include, alkylcelluloses, such as, methyl or ethyl cellulose and, hydroxyalkylcelluloses, such as hydroxymethyl cellulose, hydroxyethyl cellulose, hydroxypropyl cellulose, hydroxypropylmethyl cellulose, hydroxymethylpropyl cellulose, and combinations thereof. Useful alkylcelluloses include those sold under the trade names Methocel E™ by Dow Chemicals. Additionally, useful ethylcelluloses are commercially available commercially available from FMC Corporation under brand name Aquacoat ECD. These acrylic polymers are generally water soluble materials.

Moreover, the pharmaceutically active agents may be sprayed and congealed with fully hydrogenated oils or waxes considered safe for human consumption and are relatively stable. Useful, but non-limiting, pharmaceutically acceptable oils include mineral oil, peanut oil, soybean oil, sunflower oil, corn oil, olive oil, hard palm oil and rapeseed oil.

Furthermore, crown ether compounds, such as cyclodextrins, are also useful for coating the pharmaceutically active agents. The pharmaceutically active agents are taste masked with crown ethers through entrapment or coaccervation methods. Useful cyclodextrins are commercially available under the trade name of Trappsol® from CTD, Inc.

Pharmaceutically active agents may be taste masked with the above-described taste-masking agents by a variety of techniques. The techniques coat the pharmaceutically active agents or portions of the pharmaceutically active agents with taste masking agents to avoid the unpleasant taste effects, such as bitterness, often associates with the pharmaceutically active agents or drugs. Useful coating techniques include, but are not limited to, fluidized bed coating, spray congealing coating, agglomeration or granulation coating, entrapment coating, coaccervation coating, infusion coating, spin coating, ion exchange coating and the like.

The fluidized bed coating method is commonly used in pharmaceutical industries for taste masking pharmaceutically active agents. Fluidized bed coaters achieve fluidization of the pharmaceutically active agents by introducing a continuous stream of process gas into a chamber. The coating material is deposited onto the suspended agent as it passes through the spray path of the coating material. The coated agents are dried. A relative low water solubility polymer is typically used to coat the active particles' surface. Minimum limits on particle sizes are about 100 to 120 microns. Smaller particle sizes are difficult to achieve due to process limitation and product loss. Water insoluble pharmaceutically active agents may be suitably coated with water soluble taste masking agents with this method.

In the spray congealing method both the pharmaceutically active agents and the coating materials are sprayed simultaneously into a chamber supplied with process gas to create a uniformly coated active. This method typically involves the coating of the actives with material that could be melted at reasonable temperatures, for example fatty materials or polymers such as certain Eudragit® polymers. The mix of materials are sprayed through a fine nozzle and cooled through a temperature-control air stream or a cold surface. Consideration of mixture temperature is important. The melting temperature of the coating agent selected should not exceed a degradation temperature of the pharmaceutically active agent.

In the agglomeration or granulation method, the pharmaceutically active agents are mixed with the taste-masking agents and a solvent by mechanical means or by spray drying. The solvent is gradually removed by vacuum or heating, or both. Particles are then agglomerated. The agglomerated particles are not typically coated entirely with the taste masking agent and some bitterness may result accordingly. The bitterness, however, may be further reduced by incorporating such coated particles in the films described herein.

In typical entrapment coating methods, certain compounds having specific properties that can trap pharmaceutically active agents into its molecule cages must first be selected. Compounds, like certain specifically made starches and crown ether type molecules, such as cyclodextrins and zeolites, are useful with this method. The compounds and the agents are entrapped by ionic attraction. The entrapped agents are then precipitated from solution.

The coaccervation coating method uses two polymers with opposite charges in solution. When the solution is neutralized an insoluble matrix will precipitate from solution and trap the pharmaceutically active agents therein. Examples include interactions of gum arabic and gelatin solutions and interactions of cyclodextrins and protein solutions.

In the infusion method pharmaceutically active agents and flavors or sweeteners are dissolved and infused into a polymer matrix to form a dry powder. In spin coating methods, pharmaceutically active agents are combined with sugars or fats and spun into coated particles. Details of the method are disclosed in U.S. Patent No. 5,028,632, the contents of which is incorporated herein by reference. In ion exchange coating, ionic bonding of pharmaceutically active agents to ion exchange resins masks the tastes of the agents.

Extrusion and spheronization methods may also be used of taste-masking pharmaceutically active particulates. Ratios of active(s) and polymer(s) (such as, starch, cellulose, gum and/or combinations thereof) are first mixed and thicken by adding a small amount of water. The thickened mixture is then extruded through a single or double nozzle screw. Small spherical particles are formed by a Marumerization® process. Desirable particle sizes are obtained through process control and particulate sieving.

Lyophilization (Freeze-Drying) methods may also be used with the practice of the present invention A combination of polymer(s) (such as, starch, gum, cellulose and/or combinations thereof) with active(s) are mixed and dissolved (or dispersed) in aqueous medium. This mixture is then freeze-dried on a pre-form substrate. Desirable particles sizes can be obtained by process control and product sieving.

In some instances, taste-masking may amount to the addition of two components together, neither of which are particularly pleasing to the taste, but which, due to their chemical makeup, counteract each other or allow for a third substance or more of one of the substances to be added without a concomitant reduction in pleasantness of the taste.

The edible water-soluble delivery system described herein further includes one or moremembersselected from antifoaming agents, plasticizing agents, surfactants, emulsifying agents, thickening agents, binding agents, cooling agents, saliva-stimulating agents, sweetening agents, antimicrobial agents, antigens and combinations thereof.

Described herein is a drug delivery composition which includes (i) a flowable water-soluble film forming matrix; (ii) a particulate bioeffecting agent uniformly stationed therein; and (iii) a taste-masking agent coated or intimately associated with the particulate to provide taste-masking of the bioeffecting agent. The combined particulate and taste-masking agent have a particle size of 200 microns or less, and the flowable water-soluble film forming matrix is capable of being dried without loss of uniformity in the stationing of the particulate bioeffecting agent therein.

Desirably, the size of the combined particulate and taste-masking agent have a particle size of 150 microns or less, for example 100 microns or less. Moreover, such particles may be spherical, substantially spherical, or non-spherical, such as irregularly shaped particles or ellipsoidally shaped particles. Ellipsoidally shaped particles or ellipsoids are desirable because of their ability to maintain uniformity in the film forming matrix as they tend to settle to a lesser degree as compared to spherical particles. Furthermore, the flowable water-soluble film forming matrix is formable into a dry film of less than about 380 microns in thickness, for example less than about 250 microns in thickness.

Desirably, taste-masking agent is a thin film coating over portions of the bioeffecting agent. Useful taste-masking agents include polymeric materials. Water-soluble polymers are also useful. Desirably, the water-soluble polymers have an average molecular weight of equal to or greater than about 40,000. Furthermore, water-soluble polymer may be acrylic polymers, cellulosic polymers, and combinations thereof. Additionally, vinyl polymers, crown ethers, hydrogenated oils and waxes, and combinations thereof may also be used as taste-masking agents.

The matrix may be a cellulosic material; a gum; a protein; a starch; a glucan; and combinations thereof; such as but not limited to carboxymethyl cellulose; methyl cellulose; ethyl cellulose; hydroxymethyl cellulose; hydroxyethyl cellulose; hydroxypropyl cellulose; hydroxypropylmethyl cellulose; hydroxymethylpropyl cellulose; gum arabic; xanthan gum; tragacanth; acacia; carageenan; guar gum; locust bean gum; pectin; alginates; gelatinized, modified or unmodified starch, including tapioca starch, rice starch, corn starch, potato starch, and wheat starch; polyvinyl alcohol; polyacrylic acid; polyvinyl pyrrolidone; poly(meth)acrylate; poly(meth)copolymers; dextrin; dextran; proteins, such as, gelatin, zein, gluten, soy protein, soy protein isolate, and whey protein; whey protein isolate; casein; levin; collagen; chitin; chitosin; polydextrose and combinations thereof.

The bioeffecting agent may be present in amounts of up to about 0.1% to about 60% by weight of the total composition. Useful bioeffecting agents include, but are not limited to, antimicrobial agents, non-steroidal anti-inflammatory drugs, anti-tussives, decongestants, antihistamines, expectorants, anti-diarrheals, $H_2$ antagonists, proton pump inhibitors, general non-selective CNS depressants, general non-selective CNS stimulants, selective CNS functional modifiers, anti-parkinsonism drugs, narcotics, analgesics, erectile dysfunction therapies, anti-pyretics, psychopharmacological drugs and combinations thereof. The delivery vehicle composition may further include an organoleptic agent.

Described herein is a drug delivery vehicle which includes (i) a water-soluble film matrix; and (ii) a particulate bioeffecting agent uniformly suspended within the matrix and having associated with it a taste-masking agent. The uniformity is determined by the presence of no more than a 10% by weight of drug variance throughout the matrix. Desirably, the drug variance is less than 5% by weight, less than 2% by weight, less than 1% by weight, or less than 0.5% by weight. Moreover, the particulates have a particle size of 200 microns or less. Furthermore, the film matrix desirably has a thickness of less than about 380 microns.

Useful taste-masking agents include water-soluble polymers. Desirably, the water-soluble polymer has an average molecular weight of equal to or greater than about 40,000. Non-limiting water-soluble polymers include acrylic polymers, cellulosic polymers, and combinations thereof. The taste-masking agents may also include vinyl polymers, crown ethers, hydrogenated oils and waxes, and combinations thereof. The drug delivery vehicle may further include an organoleptic agent with the bioeffecting agent.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent coated or encapsulated with a water-soluble polymer having an average molecular weight of equal to or greater than about 25,000. Water-soluble polymers having an average molecular weight of equal to or greater than about 40,000 are also useful. Useful water-soluble polymers include acrylic polymers, cellulosic polymers, and combinations thereof. Desirably, the pharmaceutically active particles are embedded within the film. Additionally, the film includes sections of substantially equal size and the particles are distributed in an amount that varies less than about 10% among the sections. Desirably, the size of the particles are about 200 microns or less. Desirably, the film has a thickness of less than about 380 microns. Moreover, the drug delivery vehicle may further include an organoleptic agent with the water-soluble polymer.

Described herein is a drug delivery vehicle which includes a dry mucoadhering film having a thickness defined by opposed surfaces. The film includes (i) a water-soluble polymer; and (ii) a pharmaceutically active particle having a pharmaceutically active agent and a taste-masking agent present in the amount of about 15-80% by weight of the particle. Desirably, the taste-masking agent is present in the amount of about 20-60% by weight of the particle. More desirably, the taste-masking agent is present in the amount of about 25-35% by weight of the particle. The pharmaceutically active particle is desirably embedded within the film, and the film includes sections of substantially equal size where the particles are distributed in an amount that varies less than about 10% among the sections. Useful sizes of the pharmaceutically active particles include particle sizes of 200 microns or less. Desirably, the film has a thickness of less than about 380 microns. The drug delivery vehicle may further include an organoleptic agent with the taste-masking agent.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent and a taste-masking agent. The active particle has a particle size of less than about 200 microns. Desirably, the thickness of the film is less than about 380 microns.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent and a taste-masking agent. The particle desirably has a particle size of less than about 200 microns and the taste-masking agent is present in amounts of about 15-80% by weight of the particle. A particle size of about 150 microns or less is also useful. Desirably, the particle size of the particle is about 100 microns or less. Desirably, the thickness of the film is less than about 380 microns, for example, less than about 250 microns. Furthermore, the taste-masking agent may be present in the amount of about 20-60% by weight of the particle. Desirably, the taste-masking agent is present in the amount of about 25-35% by weight of the particle.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent and an organoleptic agent. The active particle is taste-masked with a taste-masking agent. Useful organoleptic agents include flavors, sweeteners and combinations thereof.

Described herein is a drug delivery vehicle which includes a dry mucoadhering film having a thickness defined by opposed surfaces. The film includes (i) a water-soluble polymer; and (ii) a pharmaceutically active particle comprising a pharmaceutically active agent being taste-masked with a taste-masking composition comprising a water-soluble polymer and at least one of a flavor or a sweetener.

Described herein is also a method of preparing a thin film drug delivery vehicle. The method includes the steps of (a) providing a pharmaceutically active agent / taste-masking agent complex; (b) combining the complex with a water-soluble polymer and a solvent to form a mixture with uniform distribution of the complex therein; (c) casting the mixture onto a planar carrier surface to form a thin film on the carrier surface; and (d) controllably drying the thin film to form a distribution variance of the complex having less than about 10% variance throughout any given area of the thin film. The step of providing the pharmaceutically active agent with the taste-masking agent includes a treatment for coating the taste masking agent onto portions of the pharmaceutically active agent.

The drying includes applying heat to the bottom of the carrier surface. Moreover, the drying may include applying microwave energy to the film. Such microwave drying is useful because drying initiates in the middle portions of the film. The present disclosure, however, is not limited to these drying methods. Any drying method may suitably be used as long as the drying does not initiate at the top surface of the casted mixture. Such top surface drying does not typically provide desirable film uniformity.

Useful methods for providing the pharmaceutically active agent with the taste-masking agent include fluidized bed coating, spray congealing coating, agglomeration or granulation coating, entrapment coating, coaccervation coating, infusion coating, spin coating, ion exchange coating the taste masking agent onto portions of the pharmaceutically active agent.

## Uses of Thin Films

The thin films described herein are well suited for many uses. The high degree of uniformity of the components of the film makes them particularly well suited for incorporating pharmaceuticals. Furthermore, the polymers used in construction of the films may be chosen to allow for a range of disintegration times for the films. A variation or extension in the time over which a film will disintegrate may achieve control over the rate that the active is released, which may allow for a sustained release delivery system. In addition, the films may be used for the administration of an active to any of several body surfaces, especially those including mucous membranes, such as oral, anal, vaginal, ophthalmological, the surface of a wound, either on a skin surface or within a body such as during surgery, and similar surfaces.

The films may be used to orally administer an active. This is accomplished by preparing the films as described above and introducing them to the oral cavity of a mammal. This film may be prepared and adhered to a second or support layer from which it is removed prior to use, i.e. introduction to the oral cavity. An adhesive may be used to attach the film to the support or backing material which may be any of those known in the art, and is preferably not water soluble. If an adhesive is used, it will desirably be a food grade adhesive that is ingestible and does not alter the properties of the active. Mucoadhesive compositions are particularly useful. The film compositions in many cases serve as mucoadhesives themselves.

The films may be applied under or to the tongue of the mammal. When this is desired, a specific film shape, corresponding to the shape of the tongue may be preferred. Therefore the film may be cut to a shape where the side of the film corresponding to the back of the tongue will be longer than the side corresponding to the front of the tongue. Specifically, the desired shape may be that of a triangle or trapezoid. Desirably, the film will adhere to the oral cavity preventing it from being ejected from the oral cavity and permitting more of the active to be introduced to the oral cavity as the film dissolves.

Another use for the films described herein takes advantage of the films tendency to dissolve quickly when introduced to a liquid. An active may be introduced to a liquid by preparing a film in accordance with the present disclosure, introducing it to a liquid, and allowing it to dissolve. This may be used either to prepare a liquid dosage form of an active, or to flavor a beverage.

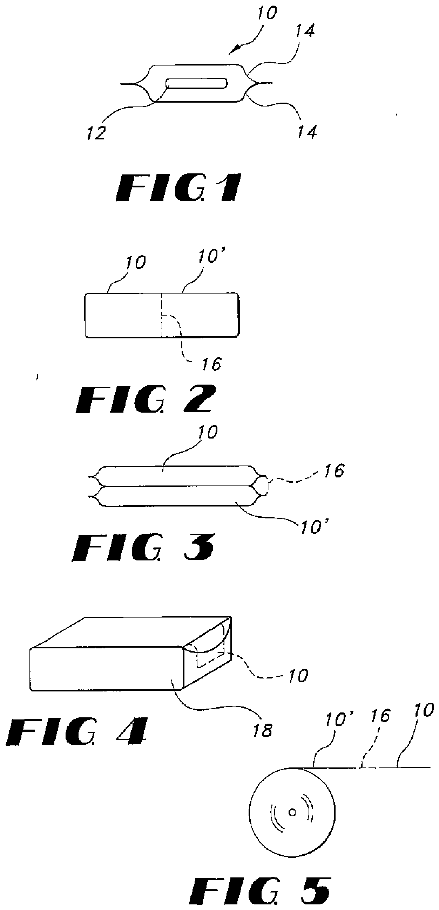

The films described herein are desirably packaged in sealed, air and moisture resistant packages to protect the active from exposure oxidation, hydrolysis, volatilization and interaction with the environment. Referring to Figure 1, a packaged pharmaceutical dosage unit 10, includes each film 12 individually wrapped in a pouch or between foil and/or plastic laminate sheets 14. As depicted in Figure 2, the pouches 10, 10' can be linked together with tearable or perforated joints 16. The pouches 10, 10' may be packaged in a roll as depicted in Figure 5 or stacked as shown in Figure 3 and sold in a dispenser 18 as shown in Figure 4. The dispenser may contain a full supply of the medication typically prescribed for the intended therapy, but due to the thinness of the film and package, is smaller and more convenient than traditional bottles used for tablets, capsules and liquids. Moreover, the films of the present disclosure dissolve instantly upon contact with saliva or mucosal membrane areas, eliminating the need to wash the dose down with water.

Desirably, a series of such unit doses are packaged together in accordance with the prescribed regimen or treatment, e.g., a 10-90 day supply, depending on the particular therapy. The individual films can be packaged on a backing and peeled off for use.

**Rheology and Films Properties**

For present purposes the term non-self-aggregating uniform heterogeneity refers to the ability of the films described herein, which are formed from one or more components in addition to a polar solvent, to provide a substantially reduced occurrence of, i.e. little or no, aggregation or conglomeration of components within the film as is normally experienced when films are formed by conventional drying methods such as a high-temperature air-bath using a drying oven, drying tunnel, vacuum drier, or other such drying equipment. The term heterogeneity, as used in the present invention, includes films that will incorporate a single component, such as a polymer, as well as combinations of components, such as a polymer and an active. Uniform heterogeneity includes the substantial absence of aggregates or conglomerates as is common in conventional mixing and heat drying methods used to form films.

Furthermore, the films described herein have a substantially uniform thickness, which is also not provided by the use of conventional drying methods used for drying water-based polymer systems. The absence of a uniform thickness detrimentally affects uniformity of component distribution throughout the area of a given film.

The film products described herein are produced by a combination of a properly selected polymer and a polar solvent, optionally including an active ingredient as well as other fillers known in the art. These films provide a non-self-aggregating uniform heterogeneity of the components within them by utilizing a selected casting or deposition method and a controlled drying process. Examples of controlled drying processes include, but are not limited to, the use of the apparatus disclosed in U.S. Patent No. 4, 631, 837 to Magoon ("Magoon"), herein incorporated by reference, as well as hot air impingement across the bottom substrate and bottom heating plates. Another drying technique for obtaining the films described herein is controlled radiation drying, in the absence of uncontrolled air currents, such as infrared and radio frequency radiation (i.e. microwaves).

The objective of the drying process is to provide a method of drying the films that avoids complications, such as the noted "rippling" effect, that are associated with conventional drying methods and which initially dry the upper surface of the film, trapping moisture inside. In conventional oven drying methods, as the moisture trapped inside subsequently evaporates, the top surface is altered by being ripped open and then reformed. These complications are avoided by the present disclosure, and a uniform film is provided by drying the bottom surface of the film first or otherwise preventing the formation of polymer film formation (skin) on the top surface of the film prior to drying the depth of the film. This may be achieved by applying heat to the bottom surface of the film with substantially no top air flow, or alternatively by the introduction of controlled microwaves to evaporate the water or other polar solvent within the film, again with substantially no top air flow. Yet alternatively, drying may be achieved by using balanced fluid flow, such as balanced air flow, where the bottom and top air flows are controlled to provide a uniform film. In such a case, the air flow directed at the top of the film should not create a condition which would cause movement of particles present in the wet film, due to forces generated by the air currents. Additionally, air currents directed at the bottom of the film should desirably be controlled such that the film does not lift up due to forces from the air. Uncontrolled air currents, either above or below the film, can create non-uniformity in the final film products. The humidity level of the area surrounding the top surface may also be appropriately adjusted to prevent premature closure or skinning of the polymer surface.

This manner of drying the films provides several advantages. Among these are the faster drying times and a more uniform surface of the film, as well as uniform distribution of components for any given area in the film. In addition, the faster drying time allows viscosity to quickly build within the film, further encouraging a uniform distribution of components and decrease in aggregation of components in the final film product. Desirably, the drying of the film will occur within about ten minutes or fewer, or more desirably within about five minutes or fewer.

The present invention yields exceptionally uniform film products when attention is paid to reducing the aggregation of the compositional components. By avoiding the introduction of and eliminating excessive air in the mixing process, selecting polymers and solvents to provide a controllable viscosity and by drying the film in a rapid manner from the bottom up, such films result.

The products and processes described herein rely on the interaction among various steps of the production of the films in order to provide films that substantially reduce the self-aggregation of the components within the films. Specifically, these steps include the particular method used to form the film, making the composition mixture to prevent air bubble inclusions, controlling the viscosity of the film forming composition and the method of drying the film. More particularly, a greater viscosity of components in the mixture is particularly useful when the active is not soluble in the selected polar solvent in order to prevent the active from settling out. However, the viscosity must not be too great as to hinder or prevent the chosen method of casting, which desirably includes reverse roll coating due to its ability to provide a film of substantially consistent thickness.

In addition to the viscosity of the film or film-forming components or matrix, there are other considerations taken into account by the present disclosure for achieving desirable film uniformity. For example, stable suspensions are achieved which prevent solid (such as drug particles) sedimentation in non-colloidal applications. One approach provided by the present disclosure is to balance the density of the particulate (pp) and the liquid phase ($\rho_1$) and increase the viscosity of the liquid phase ($\mu$). For an isolated particle, Stokes law relates the terminal settling velocity (Vo) of a rigid spherical body of radius (r) in a viscous fluid, as follows:

$$V_o = (2gr')(\rho_p - \rho_l)/9\mu$$

At high particle concentrations, however, the local particle concentration will affect the local viscosity and density. The viscosity of the suspension is a strong function of solids volume fraction, and particle-particle and particle-liquid interactions will further hinder settling velocity.

Stokian analyses has shown that the incorporation of a third phase, dispersed air or nitrogen, for example, promotes suspension stability. Further, increasing the number of particles leads to a hindered settling effect based on the solids volume fraction. In dilute particle suspensions, the rate of sedimentation, v, can be expressed as:

$$v/V_o = 1/(1 + \kappa\varphi)$$

where $\kappa$ = a constant, and $\varphi$ is the volume fraction of the dispersed phase. More particles suspended in the liquid phase results in decreased velocity. Particle geometry is also an important factor since the particle dimensions will affect particle-particle flow interactions.

Similarly, the viscosity of the suspension is dependent on the volume fraction of dispersed solids. For dilute suspensions of non-interaction spherical particles, an expression for the suspension viscosity can be expressed as:

$$\mu/\mu_o = 1 + 2.5\phi$$

where $\mu_o$ is the viscosity of the continuous phase and $\phi$ is the solids volume fraction. At higher volume fractions, the viscosity of the dispersion can be expressed as

$$\mu/\mu_o = 1 + 2.5\varphi + C_1\varphi^2 + C_2\varphi^3 + \ldots$$

where C is a constant.

The viscosity of the liquid phase is critical and is desirably modified by customizing the liquid composition to a viscoelastic non-Newtonian fluid with low yield stress values. This is the equivalent of producing a high viscosity continuous phase at rest. Formation of a viscoelastic or a highly structured fluid phase provides additional resistive forces to particle sedimentation. Further, flocculation or aggregation can be controlled minimizing particle-particle interactions. The net effect would be the preservation of a homogeneous dispersed phase.

The addition of hydrocolloids to the aqueous phase of the suspension increases viscosity, may produce viscoelasticity and can impart stability depending on the type of hydrocolloid, its concentration and the particle composition, geometry, size, and volume fraction. The particle size distribution of the dispersed phase needs to be controlled by selecting the smallest realistic particle size in the high viscosity medium, i.e., $<500\mu m$. The presence of a slight yield stress or elastic body at low shear rates may also induce permanent stability regardless of the apparent viscosity. The critical particle diameter can be calculated from the yield stress values. In the case of isolated spherical particles, the maximum shear stress developed in settling through a medium of given viscosity can be given as

$$\tau_{max} = 3V\mu/2r$$

For pseudoplastic fluids, the viscosity in this shear stress regime may well be the zero shear rate viscosity at the Newtonian plateau.

A stable suspension is an important characteristic for the manufacture of a pre-mix composition which is to be fed into the film casting machinery film, as well as the maintenance of this stability in the wet film stage until sufficient drying has occurred to lock-in the particles and matrix into a sufficiently solid form such that uniformity is maintained. For viscoelastic fluid systems, a rheology that yields stable suspensions for an extended time period, such as 24 hours, must be balanced with the requirements of high-speed film casting operations. A desirable property for the films is shear thinning or pseudoplasticity, whereby the viscosity decreases with increasing shear rate. Time dependent shear effects such as thixotropy are also advantageous. Structural recovery and shear thinning behavior are important properties, as is the ability for the film to self-level as it is formed.

The rheology requirements for the inventive compositions and films are quite severe. This is due to the need to produce a stable suspension of particles, for example 30-60 wt%, in a viscoelastic fluid matrix with acceptable viscosity values throughout a broad shear rate range. During mixing, pumping, and film casting, shear rates in the range of $10 - 10^5$ sec.$^{-1}$ may be experienced and pseudoplasticity is the preferred embodiment.

In film casting or coating, rheology is also a defining factor with respect to the ability to form films with the desired uniformity. Shear viscosity, extensional viscosity, viscoelasticity, and structural recovery will influence the quality of the film. As an illustrative example, the leveling of shear-thinning pseudoplastic fluids has been derived as

$$\alpha^{(n-1/n)} = \alpha_o^{(n-1/n)} - ((n-1)/(2n-1))(\tau/K)^{1/n} (2\pi/\lambda)^{(3+n)/n} h^{(2n+1)/n} t$$

where $\alpha$ is the surface wave amplitude, $\alpha_o$ is the initial amplitude, $\lambda$ is the wavelength of the surface roughness, and both "n" and "K" are viscosity power law indices. In this example, leveling behavior is related to viscosity, increasing as n decreases, and decreasing with increasing K.

Desirably, the films or film-forming compositions described herein have a very rapid structural recovery, i.e. as the film is formed during processing, it doesn't fall apart or become discontinuous in its structure and compositional uniformity. Such very rapid structural recovery retards particle settling and sedimentation. Moreover, the films or film-forming compositions described herein are desirably shear-thinning pseudoplastic fluids. Such fluids with consideration of properties, such as viscosity and elasticity, promote thin film formation and uniformity.

Thus, uniformity in the mixture of components depends upon numerous variables. As described herein, viscosity of the components, the mixing techniques and the rheological properties of the resultant mixed composition and wet casted film are important aspects. Additionally, control of particle size and particle shape are further considerations. Desirably, the size of the particulate a particle size of 150 microns or less, for example 100 microns or less. Moreover, such particles may be spherical, substantially spherical, or non-spherical, such as irregularly shaped particles or ellipsoidally shaped particles. Ellipsoidally shaped particles or ellipsoids are desirable because of their ability to maintain uniformity in the film forming matrix as they tend to settle to a lesser degree as compared to spherical particles.

Although a variety of different polymers may be used, it is desired to select polymers to provide a desired viscosity of the mixture prior to drying. For example, if the active or other components are not soluble in the selected solvent, a polymer that will provide a greater viscosity is desired to assist in maintaining uniformity. On the other hand, if the components are soluble in the solvent, a polymer that provides a lower viscosity may be preferred.

The polymer plays an important role in affecting the viscosity of the film. Viscosity is one property of a liquid that controls the stability of the active in an emulsion, a colloid or a suspension. Generally the viscosity of the matrix will vary from about 400 cps ("cps" or "centipoise") to about 100,000 cps, preferably from about 800 cps to about 60,000 cps, and most preferably from about 1,000 cps to about 40,000 cps. Desirably, the viscosity of the film-forming matrix will rapidly increase upon initiation of the drying process.

The viscosity may be adjusted based on the selected active depending on the other components within the matrix. For example, if the component is not soluble within the selected solvent, a proper viscosity may be selected to prevent the component from settling which would adversely affect the uniformity of the resulting film. The viscosity may be adjusted in different ways. To increase viscosity of the film matrix, the polymer may be chosen of a higher molecular weight or crosslinkers may be added, such as salts of calcium, sodium and potassium. The viscosity may also be adjusted by adjusting the temperature or by adding a viscosity increasing component. Components that will increase the viscosity or stabilize the emulsion/suspension include higher molecular weight polymers and polysaccharides and gums, which include without limitation, alginate, carrageenan, hydroxypropyl methyl cellulose, locust bean gum, guar gum, xanthan gum, dextran, gum arabic, gellan gum and combinations thereof.

**Film Component Mixing:**

A number of techniques may be employed in the mixing stage to prevent bubble inclusions in the final film. To provide a composition mixture with substantially no air bubble formation in the final product, anti-foaming or surface-tension reducing agents are employed. Additionally, the speed of the mixture is desirably controlled to prevent cavitation of the mixture in a manner which pulls air into the mix. Finally, air bubble reduction can further be achieved by allowing the mix to stand for a sufficient time for bubbles to escape prior to drying the film. Desirably, the inventive process first forms a masterbatch of film-forming components without active ingredients such as drug particles or volatile materials such as flavor oils. The actives are added to smaller mixes of the masterbatch just prior to casting. Thus, the masterbatch pre-mix can be allowed to stand for a longer time without concern for instability in drug or other ingredients.

When the matrix is formed including the film-forming polymer and polar solvent in addition to any additives and the active ingredient, this may be done in a number of steps. For example, the ingredients may all be added together or a pre-mix may be prepared. The advantage of a pre-mix is that all ingredients except for the active may be combined in advance, with the active added just prior to formation of the film. This is especially important for actives that may degrade with prolonged exposure to water, air or another polar solvent.

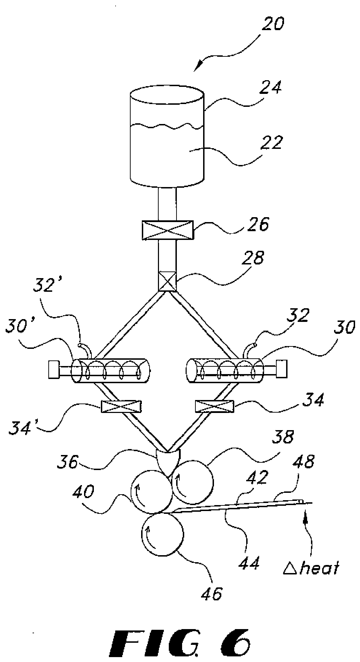

Figure 6 shows an apparatus 20 suitable for the preparation of a pre-mix, addition of an active and subsequent formation of a film. The pre-mix or master batch 22, which includes the film-forming polymer, polar solvent, and any other additives except a drug active is added to the master batch feed tank 24. The components for pre-mix or master batch 22 are desirably formed in a mixer (not shown) prior to their addition into the master batch feed tank 24. Then a pre-determined amount of the master batch is controllably fed via a first metering pump 26 and control valve 28 to either or both of the first and second mixers, 30, 30'. The present disclosure, however, is not limited to the use of two mixers, 30, 30', and any number of mixers may suitably be used. Moreover, the present disclosure is not limited to any particular sequencing of the mixers 30, 30', such as parallel sequencing as depicted in Figure 6, and other sequencing or arrangements of mixers, such as series or combination of parallel and series, may suitably be used. The required amount of the drug or other ingredient, such as a flavor, is added to the desired mixer through an opening, 32, 32', in each of the mixers, 30, 30'. Desirably, the residence time of the pre-mix or master batch 22 is minimized in the mixers 30, 30'. While complete dispersion of the drug into the pre-mix or master batch 22 is desirable, excessive residence times may result in leaching or dissolving of the drug, especially in the case for a soluble drug. Thus, the mixers 30, 30' are often smaller, i.e. lower residence times, as compared to the primary mixers (not shown) used in forming the pre-mix or master batch 22. After the drug has been blended with the master batch pre-mix for a sufficient time to provide a uniform matrix, a specific amount of the uniform matrix is then fed to the pan 36 through the second metering pumps, 34, 34'. The metering roller 38 determines the thickness of the film 42 and applies it to the application roller. The film 42 is finally formed on the substrate 44 and carried away via the support roller 46.

**Forming the Film**

The films of the present invention must be formed into a sheet prior to drying. After the desired components are combined to form a multi-component matrix, including the polymer, water, and an active or other components as desired, the combination is formed into a sheet or film, by any method known in the art such as extrusion, coating, spreading, casting or drawing the multi-component matrix. If a multi-layered film is desired, this may be accomplished by co-extruding more than one combination of components which may be of the same or different composition. A multi-layered film may also be achieved by coating, spreading, or casting a combination onto an already formed film layer.

Although a variety of different film-forming techniques may be used, it is desirable to select a method that will provide a flexible film, such as reverse roll coating. The flexibility of the film allows for the sheets of film to be rolled and transported for storage or prior to being cut into individual dosage forms. Desirably, the films will also be self-supporting or in other words able to maintain their integrity and structure in the absence of a separate support. Furthermore, the films of the present invention may be selected of materials that are edible or ingestible.

Coating or casting methods are particularly useful for the purpose of forming the films of the present invention. Specific examples include reverse roll coating, gravure coating, immersion or dip coating, metering rod or meyer bar coating, slot die or extrusion coating, gap or knife over roll coating, air knife coating, curtain coating, or combinations thereof, especially when a multi-layered film is desired.

Roll coating, or more specifically reverse roll coating, is particularly desired when forming films in accordance with the present invention. This procedure provides excellent control and uniformity of the resulting films, which is desired in the present invention. In this procedure, the coating material is measured onto the applicator roller by the precision setting of the gap between the upper metering roller and the application roller below it. The coating is transferred from the application roller to the substrate as it passes around the support roller adjacent to the application roller. Both three roll and four roll processes are common.

The gravure coating process relies on an engraved roller running in a coating bath, which fills the engraved dots or lines of the roller with the coating material. The excess coating on the roller is wiped off by a doctor blade and the coating is then deposited onto the substrate as it passes between the engraved roller and a pressure roller.

Offset Gravure is common, where the coating is deposited on an intermediate roller before transfer to the substrate.

In the simple process of immersion or dip coating, the substrate is dipped into a bath of the coating, which is normally of a low viscosity to enable the coating to run back into the bath as the substrate emerges.

In the metering rod coating process, an excess of the coating is deposited onto the substrate as it passes over the bath roller. The wire-wound metering rod, sometimes known as a Meyer Bar, allows the desired quantity of the coating to remain on the substrate. The quantity is determined by the diameter of the wire used on the rod.

In the slot die process, the coating is squeezed out by gravity or under pressure through a slot and onto the substrate. If the coating is 100% solids, the process is termed "Extrusion" and in this case, the line speed is frequently much faster than the speed of the extrusion. This enables coatings to be considerably thinner than the width of the slot.

The gap or knife over roll process relies on a coating being applied to the substrate which then passes through a "gap" between a "knife" and a support roller. As the coating and substrate pass through, the excess is scraped off.

Air knife coating is where the coating is applied to the substrate and the excess is "blown off" by a powerful jet from the air knife. This procedure is useful for aqueous coatings.

In the curtain coating process, a bath with a slot in the base allows a continuous curtain of the coating to fall into the gap between two conveyors. The object to be coated is passed along the conveyor at a controlled speed and so receives the coating on its upper face.

### Drying the Film

While the proper viscosity, uniformity in mixture and stable suspension of particles, and casting method are important in the initial steps of forming the film to promote uniformity, the method of drying the wet film is also important. Although these parameters and properties assist uniformity initially, a controlled rapid drying process ensures that the uniformity will be maintained until the film is dry. A controlled drying process is particularly important when, in the absence of a viscosity increasing composition or a composition in which the viscosity is controlled, for example by the selection of the polymer, the components within the film may have an increased tendency to aggregate or conglomerate. An alternative method of forming a film with an accurate dosage, that would not necessitate the controlled drying process, would be to cast the films on a predetermined well. With this method, although the components may aggregate, this will not result in the migration of the active to an adjacent dosage form, since each well may define the dosage unit per se.

When a controlled or rapid drying process is desired, this may be through a variety of methods. A variety of methods may be used including those that require the application of heat. The liquid carriers are removed from the film in a manner such that the uniformity, or more specifically, the non-self-aggregating uniform heterogeneity, that is obtained in the wet film is maintained.

Desirably, the film is dried from the bottom of the film to the top of the film. Substantially no air flow is present across the top of the film during its initial setting period, during which a solid, visco-elastic structure is formed. This can take place within the first few minutes, e.g. about the first ½ minute to about the first 4 minutes of the drying process. Controlling the drying in this manner, prevents the destruction and reformation of the film's top surface, which results from conventional drying methods. This is accomplished by forming the film and placing it on the top side of a surface having top and bottom sides. Then, heat is initially applied to the bottom side of the film to provide the necessary energy to evaporate or otherwise remove the liquid carrier. The films dried in this manner dry more quickly and evenly as compared to air-dried films, or those dried by conventional drying means. In contrast to an air-dried film that dries first at the top and edges, the films dried by applying heat to the bottom dry simultaneously at the center as well as at the edges. This also prevents settling of ingredients that occurs with films dried by conventional means.

The temperature at which the films are dried is about 100°C or less, desirably about 90°C or less, and most desirably about 80°C or less.

Another method of controlling the drying process, which may be used alone or in combination with other controlled methods as disclosed above includes controlling and modifying the humidity within the drying apparatus where the film is being dried. In this manner, the premature drying of the top surface of the film is avoided.

A specific example of an appropriate drying method is that disclosed by Magoon. Magoon is specifically directed toward a method of drying fruit pulp. However, the present inventors have adapted this process toward the preparation of thin films.

The method and apparatus of Magoon are based on an interesting property of water. Although water transmits energy by conduction and convection both within and to its surroundings, water only radiates energy within and to water. Therefore, the apparatus of Magoon includes a surface onto which the fruit pulp is placed that is transparent to infrared radiation. The underside of the surface is in contact with a temperature controlled water bath. The water bath temperature is desirably controlled at a temperature slightly below the boiling temperature of water. When the wet fruit pulp is placed on the surface of the apparatus, this creates a "refractance window." This means that infrared energy is permitted to radiate through the surface only to the area on the surface occupied by the fruit pulp, and only until the fruit pulp is dry. The apparatus of Magoon provides the films described herein with an efficient drying time reducing the instance of aggregation of the components of the film.

The films may initially have a thickness of about 500 $\mu$m to about 1,500 $\mu$m, or about 20 mils to about 60 mils, and when dried have a thickness from about 3 $\mu$m to about 250 $\mu$m, or about 0.1mils to about 10mils. Desirably, the dried films will have a thickness of about 2 mils to about 8 mils, and more desirably, from about 3 mils to about 6 mils.

The wet film is then dried using controlled bottom drying or controlled microwave drying, desirably in the absence of external air currents or heat on the top (exposed) surface of the film 48 as described herein. Controlled bottom drying or controlled microwave drying advantageously allows for vapor release from the film without the disadvantages of the prior art. Conventional convection air drying from the top is not employed because it initiates drying at the top uppermost portion of the film, thereby forming a barrier against fluid flow, such as the evaporative vapors, and thermal flow, such as the thermal energy for drying. Such dried upper portions serve as a barrier to further vapor release as the portions beneath are dried, which results in non-uniform films. As previously mentioned some top air flow can be used to aid the drying of the films described herein, but it must not create a condition that would cause particle movement or a rippling effect in the film, both of which would result in non-uniformity. If top air is employed, it is balanced with the bottom air drying to avoid non-uniformity and prevent film lift-up on the carrier belt. A balance top and bottom air flow may be suitable where the bottom air flow functions as the major source of drying and the top air flow is the minor source of drying. The advantage of some top air flow is to move the exiting vapors away from the film thereby aiding in the overall drying process. The use of any top air flow or top drying, however, must be balanced by a number of factors including, but not limited, to rheological properties of the composition and mechanical aspects of the processing. Any top fluid flow, such as air, also must not overcome the inherent viscosity of the film-forming composition. In other words, the top air flow cannot break, distort or otherwise physically disturb the surface of the composition. Moreover, air velocities are desirably below the yield values of the film, i.e., below any force level that can move the liquids in the film-forming compositions. For thin or low viscosity compositions, low air velocity must be used. For thick or high viscosity compositions, higher air velocities may be used. Furthermore, air velocities are desirably low so as to avoid any lifting or other movement of the film formed from the compositions.

Moreover, the films of the present invention may contain particles that are sensitive to temperature, such as flavors, which may be volatile, or drugs, which may have a low degradation temperature. In such cases, the drying temperature may be decreased while increasing the drying time to adequately dry the uniform films described herein. Furthermore, bottom drying also tends to result in a lower internal film temperature as compared to top drying. In bottom drying, the evaporating vapors more readily carry heat away from the film as compared to top drying which lowers the internal film temperature. Such lower internal film temperatures often result in decreased drug degradation and decreased loss of certain volatiles, such as flavors.

Furthermore, particles or particulates may be added to the film-forming composition or matrix after the composition or matrix is cast into a film. For example, particles may be added to the film 42 prior to the drying of the film 42. Particles may be controllably metered to the film and disposed onto the film through a suitable technique, such as through the use of a doctor blade (not shown) which is a device which marginally or softly touches the surface of the film and controllably disposes the particles onto the film surface. Other suitable, but non-limiting, techniques include the use of an additional roller to place the particles on the film surface, spraying the particles onto the film surface, and the like. The particles may be placed on either or both of the opposed film surfaces, i.e., the top and/or bottom film surfaces.

Desirably, the particles are securably disposed onto the film, such as being embedded into the film. Moreover, such particles are desirably not fully encased or fully embedded into the film, but remain exposed to the surface of the film, such as in the case where the particles are partially embedded or partially encased.

The particles may be any useful organoleptic agent, cosmetic agent, pharmaceutical agent, or combinations thereof. Desirably, the pharmaceutical agent is a taste-masked or a controlled-release pharmaceutical agent. Useful organoleptic agents include flavors and sweeteners. Useful cosmetic agents include breath freshening or decongestant agents, such as menthol, including menthol crystals.

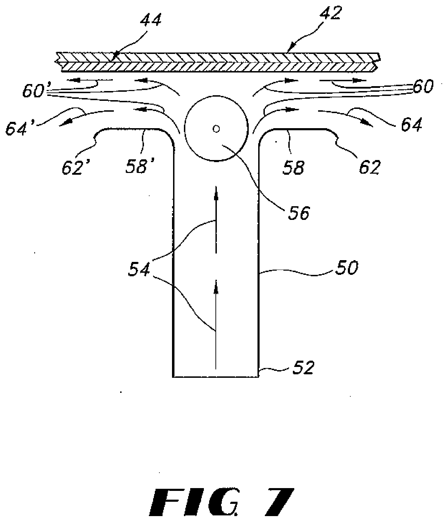

Although the inventive process is not limited to any particular apparatus for the above-described desirable drying, one particular useful drying apparatus 50 is depicted in Figure 7. Drying apparatus 50 is a nozzle arrangement for directing hot fluid, such as but not limited to hot air, towards the bottom of the film 42 which is disposed on substrate 44. Hot air enters the entrance end 52 of the drying apparatus and travels vertically upward, as depicted by vectors 54, towards air deflector 56. The air deflector 56 redirects the air movement to minimize upward force on the film 42. As depicted in Figure 7, the air is tangentially directed, as indicated by vectors 60 and 60', as the air passes by air deflector 56 and enters and travels through chamber portions 58 and 58' of the drying apparatus 50. With the hot air flow being substantially tangential to the film 42, lifting of the film as it is being dried is thereby minimized. While the air deflector 56 is depicted as a roller, other devices and geometries for deflecting air or hot fluid may suitably be used. Furthermore, the exit ends 62 and 62' of the drying apparatus 50 are flared downwardly. Such downward flaring provides a downward force or downward velocity vector, as indicated by vectors 64 and 64', which tend to provide a pulling or drag effect of the film 42 to prevent lifting of the film 42. Lifting of the film 42 may not only result in non-uniformity in the film or otherwise, but may also result in non-controlled processing of the film 42 as the film 42 and/or substrate 44 lift away from the processing equipment.

Monitoring and control of the thickness of the film also contributes to the production of a uniform film by providing a film of uniform thickness. The thickness of the film may be monitored with gauges such as Beta Gauges. A gauge may be coupled to another gauge at the end of the drying apparatus, i.e. drying oven or tunnel, to communicate through feedback loops to control and adjust the opening in the coating apparatus, resulting in control of uniform film thickness.

The film products are generally formed by combining a properly selected polymer and polar solvent, as well as any active ingredient or filler as desired. Desirably, the solvent content of the combination is at least about 30% by weight of the total combination. The matrix formed by this combination is formed into a film, desirably by roll coating, and then dried, desirably by a rapid and controlled drying process to maintain the uniformity of the film, more specifically, a non-self-aggregating uniform heterogeneity. The resulting film will desirably contain less than about 10% by weight solvent, more desirably less than about 8% by weight solvent, even more desirably less than about 6% by weight solvent and most desirably less than about 2%. The solvent may be water, a polar organic solvent including, but not limited to, ethanol, isopropanol, acetone, methylene chloride, or any combination thereof.

It has also been unexpectedly discovered that high temperature fat materials, e.g. M.P. 55°C or greater, can be used to encapsulate dry particles before or after enteric coating. The drying process temperatures are sufficiently rapid and low, and evaporative cooling effect as a result of water vapor loss is sufficiently high enough, that the fat does not appreciably melt.

Consideration of the above discussed parameters, such as but not limited to rheology properties, viscosity, mixing method, casting method and drying method, also impact material selection for the different components of the present invention. Furthermore, such consideration with proper material selection provides the compositions, including a pharmaceutical and/or cosmetic dosage form or film product having no more than a 10% variance of a pharmaceutical and/or cosmetic active per unit area. In other words, the uniformity of the present invention is determined by the presence of no more than a 10% by weight of pharmaceutical and/or cosmetic variance throughout the matrix. Desirably, the variance is less than 5% by weight, less than 2% by weight, less than 1% by weight, or less than 0.5% by weight.

The following non-limiting examples are intended to further illustrate the present invention.

## EXAMPLES

### Preparation Of Taste-Masked Pharmaceutically Active Agents:

The following drugs were coated with taste masking components and were used in the films of the present invention.

a. Fluidized Bed Coating: A taste-masked particle was prepared having a core material of northindrone (Norlutin®). Northindrone was first sieved through a 60 mesh screen having a 250 micron sieve opening. The resulting particles, i.e., having particles sizes of less than 250 microns, were then coated by the fluidized bed coating procedure in a Verse Glatt Fluidized Bed using a Wurster Column. Accordingly, a 625 grams of 5 % methylcellulose and 0.5 % Acesulfame® K (a non-caloric sweetener) solution was prepared. The solution was then applied onto 500 grams of the sieved northindrone powder at an air pressure of 40 psi through a Gustav Schlick nozzle model 941. The fluidized bed temperature was heated and maintained at 115°F during the spraying process. At the end of coating, the resulting particles were further dried therein for 3 minutes. A total of 530 grams taste masked northindrone was obtained.

b. Agglomeration Process: A sweetener solution of 94 grams of 2.5 % sodium saccharin and 2.5 % Acesulfame® K was prepared. A dry blend of 60 grams of hydroxypropylmethyl cellulose and 40 grams of silica dioxide with 20 grams polythiazide (Renese®) was made. The sweetener solution was then sprayed a little at a time onto the dry blend powder during low-shear mixing. The dry powder was, at this point, being agglomerated through the granulation/absorption process. The wet mixture was then dried in a convection oven at 105°F for 17 hours. The resulting dried product was ground in a Fitz Hammer Mill grinder and sieved through a 100 mesh screen having a 149 micron sieve opening.

c. Pelletization Process: The following product was made using a model RV02 Mix Pelletizer (made by Eirich Machines Ltd.) at maximum mixing speed. A small of crushed ice was added, slowly through a funnel, to the 40 grams Loratidine®, 40 grams Aspartame®, 10 grams hydroxypropyl cellulose and 5 grams gum arabic powder mix in the mixer while mixing at low settings of both pan rotation and mixing motor. It took 1 to 2 minutes to add the ice. Once the ice addition was completed, both the pan and the rotor mix were turned to high speed to form spherical particles. The end point was determined by examining the particles using a low power microscope. When the end point is not reached after 2 minutes of intense mixing, additional 1 to 2 minutes mixing with or without adding more ice is tried. This procedure is repeated until the end point is reach, i.e., the spherical particles are formed. The wet samples obtained were dried in a tray dryer at 55°C for about 5 hours. The resulting particles size ranged from 20 to 200 mesh. The particles were then sieved to obtain the desired particle size.

d. Infusion Method: A dry blend of 3.7 grams of Sucralose®, 10 grams fluoxetine HCl (Prozac®), and 1.25 grams polyvinylpyrrolidone were mixed uniformly. Water of 5.0 grams and 2.74 grams of propylene glycol were then added to the mixture and mixed thoroughly. To this mixture, 22 grams of hydroxypropylmethyl cellulose was added and blended under a high shear Stephan Mixer for at least 3 minutes. The resulting particles were sieved through a 100 mesh screen and were ready to be used in film matrix solution.

e. Triglyceride Reduction Formula™ microspheres from Southwest Research Institute were coated with ethylcellulose by a spinning and congealing particle producing process. The coated particles had a particle size of less than 100 microns. The polymer condensed on the drug particles thereby imparting a taste-masked pharmaceutically active agent.

f. Tamoxifen was produced by spray coating 50 to 100 micron sized particles of Eudragit® E100 (cationic methacrylate with dimethylamino ethyl ammonium groups). During fluidized coating, coated particles were isolated using a fractional separation device which insured particles having a size of less than 150 microns. The estimated level of coating was about 15%. The polymer condensed on the drug particles thereby imparting a taste-masked pharmaceutically active agent.

g. Torsemide was coated by a critical fluid process by dissolving torsemide in polyethylene glycol (400 molecular weight) which was added to a flowing stream of supercritical $CO_2$ by using a sonic spray nozzle. The resulting droplet size was controlled to produce approximated 150 micron sized spherical particles. The particles were then moved to an apparatus used for spraying a polymer coating. The polymer condensed on the drug particles thereby imparting a taste-masked pharmaceutically active agent. The polymer coating used was Eudragit® E100 dissolved in ethanol at 15% solids. The coated product was isolated by lowering the pressure and removal of the $CO_2$ and the ethanol.

h. Felodipine was coated via an emulsion solvent evaporation method using acrylate methacrylate copolymers (Eudragit® RL or Eudragit® PO and Eudragit® RS or Eudragit® PO) as the coating materials. The mean sphere diameter was 12 microns with a drug loading of about 50%.

i. Digoxin was coated with Trappsol® cyclodextrin. A 50% (wt/vol) solution of chemically modified cyclodextrin was produced by mixing it with water at room temperature. A finely ground digoxtin (less than 15 microns) was suspended in the solution with mild stirring. The mix was stirred for 60 minutes and any undissolved drug was removed by centrifugation through a 0.45 micron sized membrane. Spray drying of the solution yielded a dry powder with a 10% drug loading.

## Preparation Of The Film Forming Composition:

A film-forming composition, Composition A in Table 1, was prepared and mixed under vacuum to remove air bubbles. In further detail, a polymer mix of hydroxypropylmethyl cellulose (Methocel™ E15), polyvinylpyrrolidone and starch and xanthan were added to water with stirring over a short period of time of about 15 minutes. The stirring was set at 350 to 1500 rpm using an axial impeller. Stirring continued for another 45 minutes after combining the components to form a viscous, uniform mix.

To this viscous mix plasticizer (propylene glycol), flavor, antifoam and sweetener were sequentially added. The mixture was stirred for an additional 10 minutes at 500 rpm before the addition of a taste-masked drug.

### TABLE 1

| Film Forming Polymer Composition | Composition |
|---|---|
| Ingredient | A |
| Hydroxypropylmethyl cellulose | 8.5 |
| Polyvinylpyrro lidone | 5.5 |
| Starch | 5.5 |
| Sweetener | 2.4 |
| Flavor (Mint Mix) | 3.3 |
| Xanthan Gum | 0.3 |
| Plasticizer | 3.4 |
| Antifoam agent | 0.8 |
| Water | 70.4 |
| Total: | 100 |

A taste-masked drug was added to the mixture in about a 5 minute time period. After the addition of the drug the mixture was placed under a vacuum from about 0.1 to about 0.7 torr for about 45 minutes.

### Film Compositions With Taste-Masked Pharmaceutically Active Agents:

After removing the vacuum, the product mix was added to a coating pan and filmed using a three-roll coater. The suspension was coated at 250 microns onto siliconized paper substrate and moved through a drying oven heated at 90°C. The composition was dried in accordance with the process set forth in co-pending U.S. Application No. 10/074,272.

The dried product was examined for physical appearance, dissolution in the mouth and bitterness.

The resultant uncut films of inventive composition A with the above-described taste-masked drugs exhibited uniformity in content particularly with respect to the tasted-masked drugs, as well as unit doses of ¾" by 1" by 5-6 mils cut therefrom. The inventive compositions also were observed to have a smooth surface, absent of air bubbles. The films had minimal taste when ingested. All films dissolved in the mouth in less than 15 seconds.

The film produced with the less than 100 micron sized taste-masked triglyceride had a loading of 20 mg per 25 mm$^2$ piece of film. The film produced with the less than 150 micron sized taste-masked tamoxifen had a loading of 10 mg per 20 mm$^2$ of film (assuming 85% active). The film produced with the less than 150 micron sized taste-masked torsemide had a loading of 10 mg per 25 mm$^2$ of film (assuming 90% active). The film produced with the taste-masked digoxin had a loading of 0.5 mg per 15 mm$^2$ of film (assuming 90% active).

### Film Compositions Free of Surfactants and/or Plasticizers

The following examples of the present invention describe films and film-forming compositions that use an ethoxylated caster oil as a surfactant, or alternatively are free of surfactants, plasticizers and/or polyalcohols. Desirably, the films or film-forming compositions are essentially free of surfactants. Moreover, the films or film-forming compositions are desirably formulated to be essentially free of surfactants. Furthermore, the films or film-forming compositions are desirably formulated to be essentially free of plasticizers. Still furthermore, the films or film-forming compositions are desirably formulated to be essentially free of polyalcohols. Moreover, the films or film-forming compositions are desirably formulated to be essentially free of surfactants and plasticizers. Furthermore, the films or film-forming compositions are desirably formulated to be essentially free of surfactants, plasticizers and polyalcohols.

### TABLE 2

| Ingredient | (parts by wt.) B |
|---|---|
| POLYMERS: | |

(continued)

| Ingredient | (parts by wt.) B |
|---|---|
| Hydroxypropylmethyl cellulose | 15.6 |
| Cornstarch[1] | 10.41 |
| Polyvinylpyrrolidone | 10.41 |
| Xanthan Gum | 1.14 |
| | |
| SURFACTANT[2]: | 2.0 |
| PLASTICIZER[3]: | 11.67 |
| | |
| ANTI-FOAM AGENT[4] | 2.44 |
| | |
| OTHER | |
| Spearmint Flavor | 10.43 |
| Loratadine (drug) | 16.62 |
| Calcium Carbonate | 5.54 |
| Sweetener | 9.36 |
| [1]Available from Grain Processing Corporation as Pure Cote B792 [2]Ethoxylated caster oil, Cremophor® EL available from BASF [3]Propylene Glycol [4]Silicone Emulsion | |

The above ingredients were added at 30% to 70% water and stirred until polymers were fully hydrated which took 45 min. The mix was then put under vacuum to eliminate entrapped air. Vacuum was added in a steady manner starting at 500 mm and progressing up to 760 mm over 45 min.

After release of the vacuum, 6 grams of the liquid was added to a coating paper using a 200 micron spiral wound rod and a K Control Coater Model 101 (RK Print Coat Inst. Ltd.). The paper substrate onto which the coating was added was a silicone coated paper. The coated paper was then dried at 90°C until about 5% moisture remained. The formula coated and dried to a film thickness of approx. 60 microns and quickly dissolved in the mouth.

### TABLE3

| Ingredient | (parts by wt.) C |
|---|---|
| POLYMERS: | |
| Hydroxypropylmethyl cellulose | 15.6 |
| Comstarch[1] | 10.41 |
| Polyvinylpyrrolidone | 10.41 |
| | |
| PLASTICIZER/SOLVENT[2]: | 22.1 |
| | |
| ANTI-FOAM AGENT[3] | 2.44 |
| | |
| OTHER | |
| Raspberry Flavor | 0.3 |

(continued)

| Ingredient | (parts by wt.) C |
|---|---|
| Calcium Carbonate[4] | 30.38 |
| Sweetener | 8.36 |

[1] Available from Grain Processing Corporation as Pure Cote B792
[2] Propylene Glycol
[3] Polydimethyl Siloxane Emulsion
[4] Functioned to mimic drug loading

The above ingredients were added to water at 40% until a homogeneous suspension was made. Vacuum was added over 20 min. starting at 500 mm Hg. and ending at 660 mm Hg. until all air was removed from suspension. Film was made as described in prior experiments. The liquid coated the silicone release substrate and dried to a uniform flexible film. The film passed the 180° bend test without cracking and dissolved in the mouth.

**TABLE 4**

| Ingredient | (parts by wt.) D |
|---|---|
| POLYMERS: | |
| Hydroxypropylmethyl cellulose | 7.8 |
| Hydroxypropyl cellulose | 7.8 |
| | |
| ANTI-FOAM AGENT[1] | 0.75 |
| | |
| OTHER | |
| Peppermint & Bittermint Flavor | 2.25 |
| Tastemasking Flavor[2] | 0.3 |
| Calcium Carbonate[3] | 15.2 |
| Sweeteners | 0.9 |

[1] Polydimethyl Siloxane Emulsion
[2] Prosweet from Virginia Dave
[3] Functioned to mimic drug loading

The above ingredients were added at 30% to 70% water and stirred until polymers were fully hydrated which took 20 min. The mix was then put under vacuum to eliminate entrapped air. Vacuum was added in a steady manner up to 760 mm over 35 min.

After release of the vacuum, the liquid was added to a coating paper using a 350 micron smooth bar and a K Control Coater Model 101 (RK Print Coat Inst. Ltd.). The paper substrate onto which the coating was added was a silicone coated paper. The coated paper was then dried at 90°C until about 4% moisture remained. The formula coated and dried to a film. The film had an acceptable taste and quickly dissolved in the mouth. The taste-masking flavor is an ingredient that affects the taste receptors to mask the receptors from registering a different, typical undesirable, taste. The film passed the 180° bend test without cracking and dissolved in the mouth.

**Claims**

1. A cast wet film delivery system as initially cast comprising;

   (i) a flowable water soluble wet film forming matrix;
   (ii) a particulate bioeffecting agent uniformly stationed within said wet matrix; and

(iii) a taste-masking agent coated onto said particulate bioeffecting agent;

wherein the thickness of said wet film matrix is 500-1500$\mu$m (20-60 mils); and
wherein the combined particulate and taste-masking agent have a particle size of 200 microns or less.

2. The cast wet film system according to claim 1, wherein said film matrix is self-supporting and provides no more than 10% variance of said particulate bioeffecting agent in dosage units of substantially the same size of said film matrix.

3. The cast wet film system according to claim 1, wherein the film after drying of the matrix has a water content of 10% or less.

4. The cast wet film system according to claim 1, wherein said bioeffecting agent is present in amounts of up to 0.1% to 60% by weight of the total composition.

5. The cast wet film system according to claim 1, wherein said taste-masking agent is a water-soluble polymer having an average molecular weight of equal to or greater than 40,000.

6. The cast wet film system according to claim 5, wherein said water-soluble polymer is selected from the group consisting of acrylic polymers, cellulosic polymers, vinyl polymers, and combinations thereof.

7. The cast wet film system according to claim 1, wherein said matrix is a cellulosic polymer, a gum, a protein, a starch, a glucan, carboxymethyl cellulose, methyl cellulose, ethyl cellulose, hydroxymethyl cellulose, hydroxyethyl cellulose, hydroxypropyl cellulose, hydroxypropylmethyl cellulose, hydroxymethylpropyl cellulose, gum arabic, xanthan gum, tragacanth, acacia, carageenan, guar gum, locust bean gum, pectin, alginates polyvinyl alcohol, polyacrylic acid, polyvinyl pyrrolidone, poly(meth)acrylate, poly(meth)copolymers, dextrin, dextran, chitin, chitosin, polydextrose, fructose oligomers and combinations thereof.

8. The cast wet film system according to claim 7, wherein said starch is gelatinized, modified or unmodified.

9. The cast wet film system according to claim 1, wherein said matrix is a protein selected from the group consisting of gelatin, zein, gluten, soy protein, soy protein isolate, whey protein, whey protein isolate, casein, levin, collagen and combinations thereof.

10. The cast wet film system according to claim 1, wherein said bioeffecting agent is selected from the group consisting of antimicrobial agents, non-steroidal anti-inflammatory drugs, anti-tussives, decongestants, antihistamines, expectorants, anti-diarrheals, H2 antagonists, proton pump inhibitors, general non-selective CNS depressants, general non-selective CNS stimulants, selective CNS functional modifiers, anti-parkinsonism drugs, narcotics, analgesics, erectile dysfunction therapies, anti-pyretics, psychopharmacological drugs, ace-inhibitors, antianginal drugs, antiarrhythmias, anti-asthmatics, anti-cholesterolemics, anesthetics, anti-convulsants, anti-depressants, anti-diabetic agents, anti-diarrhea preparations, antidotes, anti-hypertensive drugs, anti-lipid agents, anti-manics, anti-nauseants, anti-stroke agents, anti-tumor drugs, anti-viral agents, acne drugs, alkaloids, amino acid preparations, anti-uricemic drugs, anabolic preparations, systemic and non-systemic anti-infective agents, anti-neoplastics, anti-rheumatic agents, appetite stimulants, biological response modifiers, blood modifiers, bone metabolism regulators, cardiovascular agents, central nervous system stimulates, cholinesterase inhibitors, contraceptives, dietary supplements, dopamine receptor agonists, endometriosis management agents, enzymes, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids, sympatholytics, tremor preparations, urinary tract agents, vasodilators, laxatives, antacids, ion exchange resins, appetite suppressants, anti-anxiety agents, anti-ulcer agents, coronary dilators, cerebral dilators, peripheral vasodilators, psycho-tropics, stimulants, vasoconstrictors, migraine treatments, antibiotics, tranquilizers, anti-psychotics, anticoagulants, anti-thrombotic drugs, hypnotics, anti-emetics, neuromuscular drugs, hyper-and hypo-glycemic agents, thyroid and anti-thyroid preparations, diuretics, antispasmodics, terine relaxants, erythropoietic drugs, cough suppressants, mucolytics, DNA and genetic modifying drugs, and combinations thereof.

11. The cast wet film system according to claim 1, further comprising at least one solvent.

**12.** The cast wet film system according to claim 1, wherein said bioeffecting agent is selected from the group consisting of an analgesic, an antiemetic, opiate derivative, and an anti-diabetic agent.

**13.** The cast wet film system according to claim 1, wherein said film matrix has a viscosity of about 1,000 cps to about 40,000 cps.

**14.** The cast wet film system according to claim 1, wherein said film product after drying of the matrix has a thickness of about 76 $\mu$m (3 mils) to about 152 $\mu$m (6 mils).

**Patentansprüche**

**1.** Zuführungssystem für gegossenen Nassfilm in ursprünglich gegossener Form, umfassend;

   (i) eine fließfähige, wasserlösliche, einen Nassfilm bildende Matrix;
   (ii) ein partikelförmiges biologisch wirksames Mittel, das gleichförmig in der nassen Matrix angeordnet ist; und
   (iii) ein geschmacksmaskierendes Mittel, das auf das partikelförmige biologisch wirksame Mittel aufgetragen ist; wobei die Stärke der Nassfilmmatrix 500-1500 $\mu$m (20-60 mils) ist; und
   wobei das kombinierte partikelförmige und geschmacksmaskierende Mittel eine Partikelgröße von 200 Mikrometern oder weniger aufweist.

**2.** System für gegossenen Nassfilm gemäß Anspruch 1, wobei die Filmmatrix selbsttragend ist und nicht mehr als 10 % Abweichung des partikelförmigen biologisch wirksamen Mittels in Dosierungseinheiten von im Wesentlichen gleicher Größe der Filmmatrix bereitstellt.

**3.** System für gegossenen Nassfilm gemäß Anspruch 1, wobei der Film nach Trocknen der Matrix einen Wassergehalt von 10 % oder weniger aufweist.

**4.** System für gegossenen Nassfilm gemäß Anspruch 1, wobei das biologisch wirksame Mittel in Mengen von bis zu 0,1 bis 60 Gewichtsprozent der Gesamtzusammensetzung vorhanden ist.

**5.** System für gegossenen Nassfilm gemäß Anspruch 1, wobei das geschmacksmaskierende Mittel ein wasserlösliches Polymer ist, das ein durchschnittliches Molekulargewicht von gleich wie oder größer als 40.000 aufweist.

**6.** System für gegossenen Nassfilm gemäß Anspruch 5, wobei das wasserlösliche Polymer ausgewählt ist aus der Gruppe, bestehend aus Acrylpolymeren, Cellulosepolymeren, Vinylpolymeren und Kombinationen davon.

**7.** System für gegossenen Nassfilm gemäß Anspruch 1, wobei die Matrix ein Cellulosepolymer, ein Gummi, ein Protein, eine Stärke, ein Glucan, Carboxymethylcellulose, Methylcellulose, Ethylcellulose, Hydroxymethylcellulose, Hydroxyethylcellulose, Hydroxypropylcellulose, Hydroxypropylmethylcellulose, Hydroxymethylpropylcellulose, Gummi arabicum, Xanthan, Tragant, Akazie, Carageenan, Guarkernmehl, Johannisbrotkernmehl, Pektin, Alginate, Polyvinylalkohol, Polyacrylsäure, Polyvinylpyrrolidon, Poly(meth)acrylat, Poly(meth)copolymere, Dextrin, Dextran, Chitin, Chitosin, Polydextrose, Fructose-Oligomere und Kombinationen davon ist.

**8.** System für gegossenen Nassfilm gemäß Anspruch 7, wobei die Stärke gelatiniert, modifiziert oder unmodifiziert ist.

**9.** Gegossenes Nassfilmsystem gemäß Anspruch 1, wobei die Matrix ein Protein ist, ausgewählt aus der Gruppe, bestehend aus Gelatine, Zein, Gluten, Sojaprotein, Sojaproteinisolat, Molkenprotein, Molkenproteinisolat, Casein, Levin, Kollagen und Kombinationen davon.

**10.** Gegossenes Nassfilmsystem gemäß Anspruch 1, wobei das biologisch wirksame Mitteln ausgewählt ist aus der Gruppe bestehend aus antimikrobiellen Mitteln, nicht-steroidalen entzündungshemmenden Medikamenten, Antitussiva, abschwellenden Mitteln, Antihistaminika, schleimlösenden Mitteln, Antidiarrhoika, H2-Antagonisten, Protonenpumpeninhibitoren, allgemeinen nicht-selektiven ZNS-Depressiva, allgemeinen nicht-selektiven ZNS-Stimulanzien, selektiven ZNS-Funktionsmodifikatoren, Mitteln gegen Parkinson, Narkotika, Analgetika, Therapien der erektilen Dysfunktion, Antipyretika, Psychopharmaka, Ace-Hemmer, Antiangina-Mitteln, Antiarrhythmika, Antiasthmatika, Anticholesterinmittel, Anästhetika, Antikonvulsiva, Antidepressiva, Antidiabetika, Mitteln gegen Durchfall, Antidota, Antihypertonika, Antifettmittel, antimanischen Mitteln, Mitteln gegen Übelkeit, Mitteln gegen Schlaganfall, Antitumormittel, antiviralen Mitteln, Aknemitteln, Alkaloiden, Aminosäurepräparaten, Mitteln gegen Harnvergiftung, Anabolika, systemischen und nicht systemischen Antiinfektiva, Antineoplastika, Antirheumatika, Appetitanregern, biologischen Reaktionsmodifikatoren, Blutmodifikatoren, Knochenstoffwechselregulatoren, Herz-Kreislauf-Mitteln, Stimulanzien des Zentralnervensystems, Cholinesterasehemmern, Kontrazeptiva, Nahrungsergänzungsmitteln, Dopaminrezeptor-Agonisten, Endometriosemitteln, Enzyme, Fruchtbarkeitsmitteln, Magen-Darm-Mitteln, homöopathischen Mitteln, Hormonen, Mitteln zur Behandlung von Hyperkalzämie und Hypokalzämie, Immunmodulatoren, Immunsuppressiva, Migränepräparaten, Mitteln gegen Reisekrankheit, Muskelrelaxantien, Mitteln zur Behandlung von Fettleibigkeit, Osteoporosepräparate, Oxytocika, Parasympatholytika, Parasympathomimetika, Prostaglandine, Psychotherapeutika, Atmungsmitteln, Beruhigungsmittel, Mitteln zur Raucherentwöhnung, Sympatholytika, Tremorpräparate, Mitteln für die Harnwege, Vasodilatatoren, Abführmittel, Antazida, Ionenaustauscherharze, Appetitzügler, Mitteln gegen Angstzustände, Mitteln gegen Magengeschwüre, Dilatatoren der Herzkranzgefäße, Dilatatoren des Gehirns, peripheren Vasodilatatoren, Psychopharmaka, Stimulanzien, Vasokonstriktoren, Migränebehandlungen, Antibiotika, Beruhigungsmittel, Antipsychotika, Antikoagulantien, Antithrombotika, Hypnotika, Antiemetika, neuromuskulären Medikamenten, Hyper- und Hypoglykämiemittel, Schilddrüsen- und Antischilddrüsenpräparaten, Diuretika, Antispasmodika, Terinrelaxantien, Erythropoetika, Hustenmitteln, Mukolytika, DNA- und genetisch modifizierenden Arzneimitteln und deren Kombinationen.

**11.** System für gegossenen Nassfilm gemäß Anspruch 1, ferner umfassend mindestens ein Lösungsmittel.

**12.** System für gegossenen Nassfilm gemäß Anspruch 1, wobei das biologisch wirksame Mitteln ausgewählt ist aus der Gruppe, bestehend aus einem Analgetikum, einem Antiemetikum, einem Opiatderivat und einem antidiabetischen Mittel.

**13.** System für gegossenen Nassfilm gemäß Anspruch 1, wobei die Filmmatrix eine Viskosität von etwa 1000 cps bis etwa 40.000 cps aufweist.

**14.** System für gegossenen Nassfilm gemäß Anspruch 1, wobei das Filmprodukt nach Trocknen der Matrix eine Stärke von etwa 76 $\mu$m (3 mils) bis etwa 152 $\mu$m (6 mils) aufweist.

## Revendications

**1.** Un système de distribution de film humide coulé tel que coulé initialement comprenant ;

> (i) une matrice fluide hydrosoluble formant un film humide ;
> (ii) un agent bioefficace particulaire placé uniformément à l'intérieur de ladite matrice humide ; et
> (iii) un agent de masquage du goût appliqué sur ledit agent bioefficace particulaire ;

dans lequel l'épaisseur de ladite matrice de film humide est comprise entre 500 et 1500 $\mu$m (20 à 60 mils) ; et dans lequel les particules et l'agent de masquage du goût combinés ont une granulométrie de 200 microns ou moins.

**2.** Le système de film humide coulé selon la revendication 1, dans lequel ladite matrice de film est autoportante et ne fournit pas plus de 10 % de variance dudit agent bioefficace particulaire dans des unités de dosage de sensiblement la même taille que ladite matrice de film

**3.** Le système de film humide coulé selon la revendication 1, dans lequel le film après séchage de la matrice a une teneur en eau de 10 % ou moins.

**4.** Le système de film humide coulé selon la revendication 1, dans lequel ledit agent bioefficace est présent en des quantités allant jusqu'à 0,1 % à 60 % en poids de la composition totale.

**5.** Le système de film humide coulé selon la revendication 1, dans lequel ledit agent de masquage du goût est un polymère hydrosoluble ayant un poids moléculaire moyen égal ou supérieur à 40000.

**6.** Le système de film humide coulé selon la revendication 5, dans lequel ledit polymère hydrosoluble est choisi dans le groupe constitué par les polymères acryliques, les polymères cellulosiques, les polymères vinyliques, et leurs combinaisons.

**EP 2 332 523 B2**

7. Le système de film humide coulé selon la revendication 1, dans lequel ladite matrice est un polymère cellulosique, une gomme, une protéine, un amidon, un glucane, une carboxyméthylcellulose, une méthylcellulose, une éthylcellulose, une hydroxyméthylcellulose, une hydroxyéthylcellulose, une hydroxypropylcellulose, une hydroxypropylméthylcellulose, une hydroxyméthylpropylcellulose, gomme arabique, gomme xanthane, gomme adragante, acacia, carraghénane, gomme de guar, gomme de caroube, pectine, alginates, alcool polyvinylique, acide polyacrylique, polyvinylpyrrolidone, poly(méth)acrylate, poly(méth)copolymères, dextrine, dextrane, chitine, chitosane, polydextrose, oligomères de fructose et leurs combinaisons.

8. Le système de film humide coulé selon la revendication 7, dans lequel ledit amidon est gélatinisé, modifié ou non modifié.

9. Le système de film humide coulé selon la revendication 1, dans lequel ladite matrice est une protéine choisie dans le groupe constitué par la gélatine, la zéine, le gluten, la protéine de soja, l'isolat de protéine de soja, la protéine de lactosérum, l'isolat de protéine de lactosérum, la caséine, la lévine, le collagène et leurs combinaisons.

10. Le système de film humide coulé selon la revendication 1, dans lequel ledit agent bioefficace est choisi dans le groupe constitué par les agents antimicrobiens, les médicaments anti-inflammatoires non stéroïdiens, les antitussifs, les décongestionnants, les antihistaminiques, les expectorants, les antidiarrhéiques, les antagonistes H2, les inhibiteurs de la pompe à protons, les dépresseurs généraux non sélectifs du SNC, les stimulants généraux non sélectifs du SNC, les modificateurs fonctionnels sélectifs du SNC, les médicaments contre le parkinsonisme, les stupéfiants, les analgésiques, les thérapies contre la dysfonction érectile, les antipyrétiques, les médicaments psychopharmacologiques, les inhibiteurs de l'ECA, les médicaments antiangoreux, les antiarythmiques, les antiasthmatiques, les médicaments anticholestérol, les anesthésiques, les anticonvulsivants, les antidépresseurs, les agents antidiabétiques, les préparations antidiarrhéiques, les antidotes, les médicaments antihypertenseurs, les agents anti-lipidiques, les antimaniaques, les antinauséeux, les agents anti-accident vasculaire cérébral, les médicaments antitumoraux, les agents antiviraux, les médicaments contre l'acné, les alcaloïdes, les préparations d'acides aminés, les médicaments anti-uricémiques, les préparations anabolisantes, les agents anti-infectieux systémiques et non systémiques, les antinéoplasiques, les agents antirhumatismaux, les stimulants de l'appétit, les modificateurs de réponse biologique, les modificateurs sanguins, les régulateurs du métabolisme osseux, les agents cardiovasculaires, les stimulants du système nerveux central, les inhibiteurs de la cholinestérase, les contraceptifs, les compléments alimentaires, les agonistes des récepteurs de la dopamine, les agents de gestion de l'endométriose, les enzymes, les agents de fertilité, les agents gastrointestinaux, les remèdes homéopathiques, les hormones, les agents de gestion de l'hypercalcémie et de l'hypocalcémie, les immunomodulateurs, les immunosuppresseurs, les préparations contre la migraine, les traitements contre le mal des transports, les relaxants musculaires, les agents de gestion de l'obésité, les préparations contre l'ostéoporose, les ocytociques, les parasympatholytiques, les parasympathomimétiques, les prostaglandines, les agents psychothérapeutiques, les agents respiratoires, les sédatifs, les aides au sevrage tabagique, les sympatholytiques, les préparations contre les tremblements, les agents des voies urinaires, les vasodilatateurs, les laxatifs, les antiacides, les résines échangeuses d'ions, les coupe-faim, les agents anti-anxiété, les agents antiulcéreux, les dilatateurs coronaires, les dilatateurs cérébraux, les vasodilatateurs périphériques, les psychotropes, les stimulants, les vasoconstricteurs, les traitements de la migraine, les antibiotiques, les tranquillisants, les antipsychotiques, les anticoagulants, les médicaments antithrombotiques, les hypnotiques, les antiémétiques, les médicaments de blocage neuro-musculaire, les agents hyperglycémiques et hypoglycémiques, les préparations thyroïdiennes et antithyroïdiennes, les diurétiques, les antispasmodiques, les relaxants utérins, les médicaments érythropoïétiques, les antitussifs, les mucolytiques, les médicaments modifiant l'ADN et la structure génétique, et leurs combinaisons.

11. Le système de film humide coulé selon la revendication 1, comprenant en outre au moins un solvant.

12. Le système de film humide coulé selon la revendication 1, dans lequel ledit agent bioefficace est choisi dans le groupe constitué d'un analgésique, d'un antiémétique, d'un dérivé opiacé, et d'un agent antidiabétique.

13. Le système de film humide coulé selon la revendication 1, dans lequel ladite matrice de film a une viscosité d'environ 1000 cps à environ 40000 cps.

14. Le système de film humide coulé selon la revendication 1, dans lequel ledit produit de film après séchage de la matrice a une épaisseur d'environ 76 $\mu$m (3 mils) à environ 152 $\mu$m (6 mils).

FIG 1

FIG 2

FIG 3

FIG 4

FIG 5

**FIG 6**

**FIG 7**

**REFERENCES CITED IN THE DESCRIPTION**

**Patent documents cited in the description**

- US 4136145 A, Fuchs
- WO 0042992 A
- WO 0170194 A
- US 074272
- US 5028632 A
- US 4631837 A, Magoon